# EUROPÄISCHE PATENTSCHRIFT

(11) **EP 1 373 237 B1**
(45) Veröffentlichungstag und Bekanntmachung des Hinweises auf die Patenterteilung: **02.02.2011**
(21) Anmeldenummer: 02727511.4
(22) Anmeldetag: 28.03.2002
(51) Int. Cl.: C07D 303/48, C07D 207/16

(54) **VERFAHREN ZUR HERSTELLUNG VON OXIRANCARBONSÄUREN UND DERIVATEN DAVON**
METHODS FOR PRODUCING OXIRANE CARBOXYLIC ACIDS AND DERIVATIVES THEREOF
PROCEDES DE PRODUCTION D'ACIDES CARBOXYLIQUE D'OXIRANE

(30) Priorität: 30.03.2001 DE 10115938
(43) Veröffentlichungstag der Anmeldung: 02.01.2004
(73) Patentinhaber: MediGene Aktiengesellschaft, 82152 Martinsried (DE)
(72) Erfinder: CERNERUD, Magnus, S-111 52 Stockholm (SE); BERNTSSON, Kristina, S-692 93 Kumla (SE)
(74) Vertreter: Bösl, Raphael Konrad
(86) Internationale Anmeldenummer: PCT/EP2002/003581
(87) Internationale Veröffentlichungsnummer: WO 2002/079178

(56) Entgegenhaltungen:
- EP-A- 0 198 348
- WO-A-82/00643
- JP-A- 01 207 308
- US-A- 4 046 889
- US-A- 4 172 934
- US-A- 4 350 633
- US-A- 4 450 275
- US-A- 4 692 459
- DATABASE CA [Online] CHEMICAL ABSTRACTS SERVICE, COLUMBUS, OHIO, US; SUZUKI, K. ET AL: "Effects of N-methacryloyl amino acid applications on hybrid layer formation at the interface of intertubular dentin" retrieved from STN Database accession no. 130:172936 XP002207428 & JOURNAL OF DENTAL RESEARCH (1998), 77(11), 1881-1888 ,
- DATABASE CA [Online] CHEMICAL ABSTRACTS SERVICE, COLUMBUS, OHIO, US; WANG, GARY T. ET AL: "Synthesis and FKBP binding of small molecule mimics of the tricarbonyl region of FK506" retrieved from STN Database accession no. 121:170041 XP002207429 & BIOORG. MED. CHEM. LETT. (1994), 4(9), 1161-6 ,
- DATABASE CA [Online] CHEMICAL ABSTRACTS SERVICE, COLUMBUS, OHIO, US; SUNJIC, VITOMIR ET AL: "Asymmetric hydrogenation of.alpha.-arylpropenoic acids catalyzed by rhodium(I) complexes of chiral ligands derived from some monosaccharides" retrieved from STN Database accession no. 112:98158 XP002207430 & GAZZ. CHIM. ITAL. (1989), 119(4), 229-33 ,
- PATENT ABSTRACTS OF JAPAN vol. 006, no. 118 (C-111), 2. Juli 1982 (1982-07-02) & JP 57 046961 A (SUMITOMO CHEM CO LTD), 17. März 1982 (1982-03-17)
- JEW S ET AL: "Asymmetric synthesis of (R)-(+)-etomoxir" TETRAHEDRON: ASYMMETRY, ELSEVIER SCIENCE PUBLISHERS, AMSTERDAM, NL, Bd. 8, Nr. 8, 24. April 1997 (1997-04-24), Seiten 1187-1192, XP004059911 ISSN: 0957-4166 in der Anmeldung erwähnt
- MARHEFKA CRAIG A ET AL: "Homology modeling using multiple molecular dynamics simulations and docking studies of the human androgen receptor ligand binding domain bound to testosterone and nonsteroidal ligands." JOURNAL OF MEDICINAL CHEMISTRY, Bd. 44, Nr. 11, 24. Mai 2001 (2001-05-24), Seiten 1729-1740, XP002207425 ISSN: 0022-2623
- KIRKOVSKY LEONID ET AL: "Chiral nonsteroidal affinity ligands for the androgen receptor. 1. Bicalutamide analogues bearing electrophilic groups in the B aromatic ring." JOURNAL OF MEDICINAL CHEMISTRY, Bd. 43, Nr. 4, 24. Februar 2000 (2000-02-24), Seiten 581-590, XP002207426 ISSN: 0022-2623
- DATABASE CROSSFIRE BEILSTEIN [Online] Beilstein Institut zur Förderung der Chemischen Wissenschaften, Frankfurt am Main, DE; Database accession no. 6114896 XP002207431 & J.CHEM.SOC, Nr. 95, 1909, Seite 561
- DATABASE CROSSFIRE BEILSTEIN [Online] Beilstein Institut zur Förderung der Chemischen Wissenschaften, Frankfurt am Main, DE; Database accession no. 80681 XP002207432 & JUSTUS LIEBIGS ANN.CHEM., Nr. 234, 1886, Seite 207
- CROUT, DAVID H. G. ET AL.: "Stereoelectronic control of the tertiary ketol rearrangement" J. CHEM. SOC., PERKIN TRANS. 2, Bd. 1, 1991, Seiten 53-62, XP002207427

## Beschreibung

Die Erfindung betrifft Verfahren zur Herstellung von Oxirancarbonsäuren und Derivaten davon, die erfindungsgemäß hergestellten Oxirancarbonsäuren und deren Verwendung in pharmazeutischen Zusammensetzungen.

Bislang wurden Oxirancarbonsäuren und Derivate davon beispielsweise zur Behandlung von Herzinsuffizienz und koronarer Herzerkrankungen eingesetzt, wie beispielsweise in der WO 95/15161 beschrieben, oder auch zur Behandlung von Diabetes (WO 82/00645). Insbesondere (+)-Etomoxir eignet sich zur Behandlung von Hyperlipämie.

In der Literatur beschriebene Verfahren zur Herstellung von Oxirancarbonsäuren und Derivaten davon sind nur im Labormaßstab durchführbar, so daß die Oxirancarbonsäuren und Derivate davon nicht in ausreichenden Mengen kostengünstig zur Verfügung gestellt werden können.

Die EP-A 0 386 654 beschreibt ein Verfahren zur Herstellung racemischer Oxirancarbonsäuren, die nach der Synthese getrennt werden müssen, um die reinen Enantiomere zu erhalten.

Ein Schlüsselschritt der Synthese von Oxirancarbonsäuren ist der Aufbau des Stereozentrums und der Ringschluß zum Oxiran. Hierfür sind in der Literatur verschiedene Verfahren beschrieben worden. So wird beispielsweise in der Veröffentlichung "Asymmetrization of 2-substituted Glycerols: Syntheses of R-Etomoxir and R-Palmoxirate" von K. Prasad et al., Tetrahedron: Asymmetry, 1990, 1, 421-424, eine Synthese ausgehend von 2-substituiertem Glycerol mit einer enantioselektiven Hydrolyse unter Verwendung von hydrolytischen Enzymen beschrieben. Die Ausbeute des Schlüsselschritts liegt bei 45%.

In der EP-B 0 046 590 wird ein Verfahren zur Herstellung von Phen(alk)oxysubstituierten Oxiranen beschrieben, bei dem das Oxiran durch Oxidation einer C-C-Doppelbindung in das Molekül eingeführt wird. Das Rohprodukt muß anschlieβend aufwendig chromatographisch gereinigt werden.

M.M.H. Crilley et al. (Tetrahedron Lett., 1989, 30, 885) beschreiben eine Synthese mit einer Sharpless-Epoxidierung mit einer Ausbeute von 49% in diesem Schlüsselschritt.

Im Artikel "Asymmetric synthesis of (R)-(+)-Etomoxir" von S. Jew et al., Tetrahedron: Asymmetry, 1997, 8, 1187-1192, ist eine Synthese über 10 Stufen beschrieben, mit der Etomoxir in Mengen von 30 mg erhalten wird. Für die Synthese wird Diazomethan verwendet, was dieses Verfahren für eine großtechnische Synthese unbrauchbar macht. Darüber hinaus wird in dieser Synthese nach dem Ringschluß zum Oxiran, das heißt nach dem Aufbau des Stereozentrums noch der Substituent an der Seitenkette variiert. Dies bedeutet, daß auch bei der weiteren Reaktionsführung die Stereochemie streng kontrolliert werden muß, um aufwendige Reinigungsschritte und Trennprobleme zu vermeiden.

Die bisher genutzten Verfahren zur Herstellung von Oxirancarbonsäuren und Derivaten davon eignen sich alle nicht für eine großtechnische Synthese, insbesondere, da teure und gefährliche Edukte oder Hilfsreagenzien eingesetzt werden und da über viele Syntheseschritte die Stereochemie kontrolliert werden muß.

Der vorliegenden Erfindung lag daher die primäre Aufgabe zugrunde, ein Verfahren zur Herstellung von Oxirancarbonsäuren und Derivaten davon bereitzustellen, bei dem eine Synthese im großtechnischen Maßstab mit kostengtinstigen Rohstoffen durchgeführt wird, die einfach und in großen Mengen zugänglich sind.

Desweiteren beschrieberen ist ein Verfahren zur Herstellung von Oxirancarbonsäuren und Derivaten davon enthaltend die Synthese einer Verbindung der allgemeinen Formel (VIII) 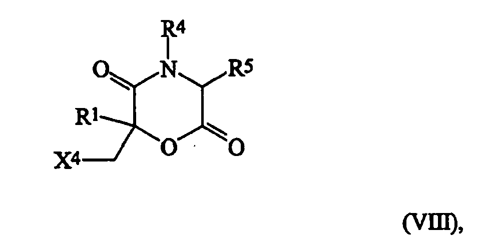 in der
- R¹ eine gerad- oder verzweigtkettige, ein- oder mehrfach substituierte oder unsubstituierte Alkylgruppe, eine gerad- oder verzweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist,
- die Reste R⁴ und R⁵ für gleiche oder verschiedene gerad- oder verzweigtkettige, ein- oder mehrfach substituierte oder unsubstituierte Alkylgruppen, gerad- oder veraweigtkettige, ein- oder mehrfach substituierte oder unsubstituierte Alkylengruppen, gerad- oder verzweigtkettige, einoder mehrfach substituierte oder unsubstituierte Aralkyl-, Alkylaryl- oder Arylgruppen stehen, wobei R⁴NCR⁵ Teil einer substituierten oder unsubstituierten cyclischen Struktur, die auch ein weiteres Heteroatom ausgewählt aus der Gruppe N, S, O enthalten kann, sein kann,
- X⁴ eine funktionelle Gruppe, die zur Ausbildung einer kationischen Zwischenstufe in einer Reaktion mit einer C-C-Doppelbindung befähigt ist und eine gute Abgangsgruppe ist, und
- nicht gleichzeitig R¹ für -(CH₂)₆-OBn steht und R⁴NCR⁵ für einen unsubstituierten Fünfring,
enthaltend die Schritte
(a) Reaktion einer Verbindung der allgemeinen Formel (V) mit einem Amin der allgemeinen Formel (VI) zu einer Verbindung der allgemeinen Formel (VII) 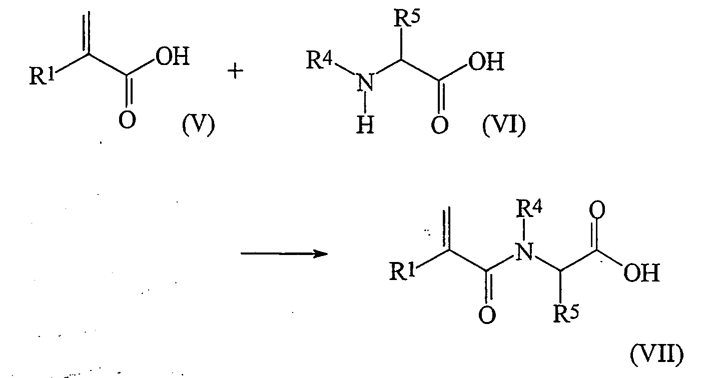 in der die Reste R¹, R⁴ und R⁵ wie oben definiert sind; und
(b) Reaktion einer Verbindung der allgemeinen Formel (VII) zu einem Lacton der allgemeinen Formel (VIII).

Das vortschend beshriebene Verfahren hat insbesondere den Vorteil, daß Oxirancarbonsäuren und Derivate davon im großtechnischen Maßstab, das heißt im Kilogramm-Maßstab, hergestellt werden können.

Unter Oxirancarbonsäuren und Derivaten davon werden im Rahmen der vorliegenden Erfindung Oxirancarbonsäuren der allgemeinen Formel (X) verstanden, sowie deren Ester mit C1- bis C15-Alkoholen und die pharmakologisch verträglichen Salze der Carbonsäuren. Als Salze kommen Salze mit anorganischen und organischen Basen in Betracht. Als Kationen für die Salzbildung werden vor allem die Kationen der Alkalimetalle, Erdalkalimetalle oder Erdmetalle verwendet. Beispielsweise seien Salze von Lithium, Natrium, Kalium, Magnesium, Calcium und Aluminium gebannt Es können jedoch auch die entsprechenden Kationen organischer Stickstoffbasen, wie Amine, Aminoalkohole, Aminozucker, basische Aminosäuren etc. eingesetzt werden. Beispielsweise sind Salze von Ethylendiamin, Dimethylamin, Diethylamin, Morpholin, Piperidin, Piperazin, N-Methylpiperazin, Methylcyclohexylamin, Benzylamin, Ethanolamin, Diethanolamin, Triethanolamin, Triseydroxymethyl)-aminoethan, 2-Amino-2-methylpropanol, 2-Amino-2-methyl-1,3-propandiol, Glucamin, N-Methylglucamin, Glucosamin, N-Methylglucosamin, Lysin, Omithin, Arginin, Chinolin zu nennen.

Für die Reaktion gemäß Schritt (a) ist es günstig, daß zunächst die Carbonsäure der allgemeinen Formel (V) aktiviert wird. Eine Aktivierung kann beispielsweise durch Bildung eines Carbonsäureanhydrids, -aktivesters oder -halo genids erfolgen. Für die technische Synthese im Rahmen des vorstdrend beschieberen verfahren ist insbesondere die Aktivierung als Carbonsäurechlorid günstig.

Eine Umsetzung der Verbindung der allgemeinen Formel (V) erfolgt mit einem geeigneten Reagenz, das zur Bildung von Carbonsäurechloriden befähigt ist, wie beispielsweise organische oder anorganische Säurechloride. Unter anderen werden Oxalylchlorid, PCl₅, PCl₃ und Thionylchlorid verwendet. Die Reaktion kann mit oder ohne organisches Lösemittel, beispielsweise unter Zusatz von Dimethylformamid (DMF) bei Temperaturen von 0 bis 60°C, beispielweise 15 bis 50°C, besonders günstig bei 20 bis 30°C durchgeführt werden. Reaktionszeiten betragen typischerweise 0,5 bis 3, beispielweise 1 bis 2 Stunden.

Die Reaktion der vorteilhafteweise aktivierten Carbonsäure mit dem Amin der allgemeinen Formel (VI) wird im basischen in polaren Lösemitteln wie Wasser, Alkoholen, Ketonen oder Ethern oder auch in Gemischen verschiedener Lösemittel, beispielweise in Gemischen aus Wasser und Aceton, Wasser und Methanol oder Wasser und Ethanol durchgeführt. Zum Einstellen des gewünschten pH-Werts werden beispielweise anorganische Basen wie Alkalihydroxide oder -alkoholate, wie Kalium- oder Natriumhydroxid verwendet. Die Reaktion wird bei Temperaturen von -10 bis 40°C, 0 bis 20°C durchgeführt. Eine typische Reaktionszeit liegt bei bis zu 1,5, insbesondere 0,5 bis 1 Stunde.

Für die Reaktion gemäß Schritt (b) wird ein Reagenz verwendet, das eine Spezies freisetzten kann, die zur Ausbildung einer kationischen Zwischenstufe mit einer Doppelbindung, wie einer C-C-Doppelbindung, fähig ist und gleichzeitig eine gute Abgangsgruppe bildet. Die mit der Doppelbindung gebildete kationische Zwischenstufe kann dann von einem Nucleophil, unteilhafteweise intramolekular, angegriffen werden, so daß ein Lacton der allgemeinen Formel (VIII) entsteht.

Als Reagenzien für eine derartige Umsetzung eignen sich beispielweise solche, die Halogen-Kationen freisetzen, wie N-Bromsuccinimid, N-Iodsuccinimid oder N-Chlorsuccinimid. Die Reaktion wird vorteihafteweise in polaren organischen Lösemitteln, beispielweise Alkoholen wie Ethanol, Methanol oder Propanol, oder auch in Lösemittelgemischen unter Zusatz von DMF durchgeführt. Der pH-Wert liegt dabei über 7. Dies wird vortelhafteweise durch die Zugaben anorganischer Alkalisalze, beispielweise Hydroxide oder Alkoholate wie Natriumhydroxid, Kaliumhydroxid oder Kalium-tert-Butoxid erreicht. Die Reaktion wird typischerweise bei Temperaturen von -10 bis 10°C, beispielweise bei weniger als 5°C, für 10 bis 24, beispielweise 12 bis 17 Stunden durchgeführt. Vorsteilhafteweise das Produkt der Reaktion durch Kristallisation gereinigt.

Im Rahmen der vorstdrend besdrieberen synthese ist es auch vorgesehen, daß die Reaktion gemäß Schritt (b) unter Kontrolle der Stereochemie durchgeführt wird.

Der Rest R¹ kann im Rahmen der vorstchend besdrieberen synthese eine Struktur der allgemeinen Formel (1) aufweisen

-(CR'R")ₙ-Zₘ-Ar (1),

in der
- Ar für einen substituierten oder unsubstituierten Phenylrest, einen substituierten oder unsubstituieren 1- oder 2-Naphtylrest oder einen substituierten oder unsubstituierten heterocyclischen Ring, beispielweise mit 5 Gliedern und aus der Gruppe Thiophen, Thiazol, Isothiazol, Pyrrol und besonders günstig Pyrazol, steht,
- R' und R" für Wasserstoff oder Fluor oder einen Methylrest steht,
- Z für -P(CR'R")ₒ- mit P gleich Sauerstoff oder Schwefel und o einer ganzen Zahl von 0 bis 4 steht, beispielweise für 1 oder 2,
- m eine ganze Zahl von 0 bis 2 ist, beispielweise 0 oder 1, und
- n eine ganze Zahl von 2 bis 8 ist, beispielweise eine ganze Zahl von 4 bis 6.

Der Rest R¹ ist unter anderem ein Rest mit 1 bis 20 Kohlenstoffatomen, beispielweise mit Halogensubstituenten, wie Fluor. Es ist weiterhin beschrieben, daß R¹ eine Struktur der allgemeinen Formel (1a) hat 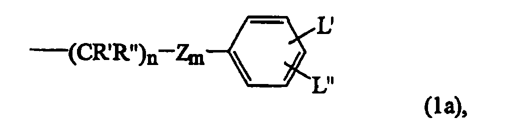 in der
- R' und R" für Wasserstoff oder Fluor oder einen Methylrest steht,
- L' und L" unabhängig voneinander für Wasserstoff, Halogen, eine substituierte oder unsubstituierte, verzweigt- oder geradkettige Alkyl-, Aryloder Alkylarylgruppe, eine substituierte oder unsubstituierte, verzweigtoder geradkettige Alkoxy- oder Aryloxygruppe, eine substituierte oder unsubstituierte, verzweigt- oder geradkettige Carboxyalkyl- oder Carboxyarylgruppe, eine Nitrogruppe oder eine Trifluormethylgruppe stehen, und
- Z für -P(CR'R")ₒ- mit P gleich Sauerstoff oder Schwefel und o einer ganzen Zahl von 0 bis 4 steht, beispielweise für 1 oder 2,
- m eine ganze Zahl von 0 bis 2 ist, beispielweise 0 oder 1, und
- n eine ganze Zahl von 2 bis 8 ist, beispielweise eine ganze Zahl von 4 bis 6.

Unter anderen steht L' oder L" für Wasserstoff und der jeweils andere Substituent L'oder L" für ein Halogenatom, beispielweise Chlor oder Fluor, vorteilhafteweise Chlor. In einer weiteren Ausführungsform sind m und o gleich 1, n ist 6 und P steht für Sauerstoff.

Der Rest X⁴, ist beispielweise ein Halogenatom, ausgewählt aus der Gruppe Chlor, Brom und Iod, vorteilhafteweise Brom.

Die Reste R⁴ und R⁵ stehen untes anderen für Alkylreste mit 1 bis 10 Kohlenstoffatomen, beispielweise 1 bis 6 Kohlenstoffatomen, die vorteilhafteweise unsubstituiert oder Fluor-substituiert sind. Im Rahmen der vorstchend beschriebenen synthese können R⁴ und R⁵ auch beispielsweiseTeil einer substituierten oder unsubstituierten cyclischen Struktur R⁴NCR⁵, die auch ein weiteres Heteroatom ausgewählt aus der Gruppe N, S, O enthalten kann, sein, wie eines Fünf- oder Sechsrings.

Bei dem Amin der allgemeinen Formel (VI) handelt es sich beispielsweise um cyclische Amine mit 5 oder 6 Ringatomen, wie um Prolin oder ein Derivat davon. In einer besonders günstigen Ausführungsform der vorschend beschriebenen synthese weist das Amin der allgemeinen Formel (VI) ein Stereozentrum auf, besonders günstig ist L-Prolin als Amin der allgemeinen Formel (VI).

In einer weiteren Ausführungsform behandelt die vorschend beschriebenen synthese weist das ein Verfahren, bei dem die Verbindung der allgemeinen Formel (V) durch eine Reaktion einer Verbindung der allgemeinen Formel (IV) erhalten wird, 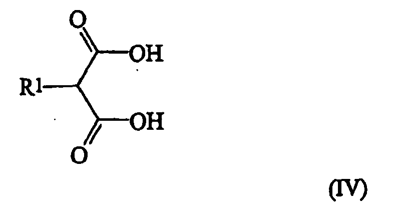 wobei in der allgemeinen Formel (IV) R¹ eine gerad- oder verzweigtkettige, einoder mehrfach substituierte oder unsubstituierte Alkylgruppe, eine gerad- oder verzweigtkettige, ein- oder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist.

Es handelt sich hierbei um eine Reaktion der Carbonsäure mit einem Reagenz, die zur Reduktion unter Ausbildung einer C-C-Doppelbindung führt. Geeignet sind hier solche Reagenzien, die nur eine der Säurefunktionen angreifen. Beispielsweise kann die Reaktion mit Formaldehyd, insbesondere Paraformaldehyd, und einer organischen Base, wie beispielsweise Triethylamin, Diisopropylethylamin oder Piperidin, insbesondere Piperidin, in organischen Lösemitteln wie Alkoholen, insbesondere Methanol, Ethanol, Propanol oder Gemischen davon, insbesondere in i-Propanol, durchgeführt werden. Die Reaktion wird bei Temperaturen von 40 bis 60°C, beispielweise bei 45 bis 55°C so lange fortgesetzt, bis der Anteil an nicht umgesetztem Edukt unter 2% laut HPLC liegt, beispielweise unter 1%, besonders günstig unter 0,5%.

In einer weiteren Ausführungsform behandelt die vorstchend beschriebenen synthese ein Verfahren, bei dem die Verbindung der allgemeinen Formel (IV) durch Hydrolyse der Verbindung der allgemeinen Formel (III) erhalten wird, 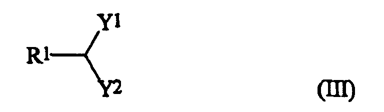 in der
- R¹ eine gerad- oder verzweigtkettige, ein- oder mehrfach substituierte oder unsubstituierte Alkylgruppe, eine gerad- oder verzweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist, und
- Y¹ und Y² gleiche oder verschiedene elektronenziehende Gruppen sind, die in eine Carbonsäure umwandelbar sind.

Die Reste Y¹ und Y² werden in diesem zuzammenhang beispielweise ausgewählt aus der Gruppe CN, Carbonsäure, Carbonsäureanhydrid und Carbonsäureester mit einem C1- bis C10-Alkohol. Bei den Resten Y¹ und Y² handelt es sich im Rahmen der unstehend beschriebenen synthese unter anderen um Reste der allgemeinen Struktur -CO₂R² und -CO₂R³, wobei R² und R³ für einen ein- oder mehrfach substituierten oder unsubstituierten Alkylrest mit 1 bis 20, unter anderem 1 bis 10, baspielweise 1 bis 4 Kohlenstoffatomen steht, vorteilhaftesweise unsubstituiert oder Fluor-substituiert.

Für diese Hydrolyse kommen alle dem Fachmann bekannten Methoden in Frage, wie saure oder basische Hydrolyse. Im Rahmen der vorstchend beschriebenen synthese ist die basische Hydrolyse unter Verwendung von Alkali-Hydroxid oder- Alkoholat, buspielsweise von Natriumhydroxid oder Kaliumhydroxid, in organisehen polaren Lösemitteln, wie Alkoholen, Ketonen oder Ethern, beispielsweise cyclischen Ethern, oder in Wasser oder in Gemischen von zwei oder mehr davon wie beispielsweise Methanol/Wasser, Tetrahydrofuran/Wasser, oder Ethanol/Wasser, gegebenenfalls unter Verwendung von Phasentransfer-Katalysatoren, günstig Die Reaktion wird vorteilhaftesweise Temperaturen von 10 bis 60 °C, unter anderem 40 bis 50 °C, besonders günstig bei 20 bis 30°C für 1 bis 24, buspelweise 1 bis 3 Stunden durchgeführt.

Eine weitere Ausftihrungsform der vorstchend beschriebenen synthese beinhaltet ein Verfahren, bei dem die Verbindung der allgemeinen Formel (III) durch Kondensation einer Verbindung der allgemeinen Formel (I) mit einer Verbindung der allgemeinen Formel (II) erhalten wird, 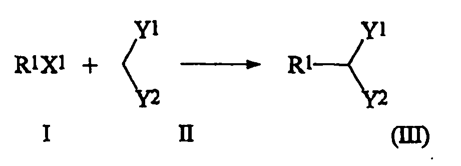 wobei
- R¹ eine gerad- oder verzweigtkettige, ein- oder mehrfach substituierte oder unsubstituierte Alkylgruppe, eine gerad- oder verzweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist,
- X¹ eine Abgangsgruppe ist,
- Y¹ und Y² gleiche oder verschiedene elektronenziehende Gruppen sind, die in eine Carbonsäure umwandelbar sind.

Der Substituent X¹ ist im Rahmen der vorstchend beschriebenen synthese beispielsweise ein Halogenatom, eine Tosylatgruppe oder eine Triflatgruppe, unter anderem Chlor oder Brom.

Die Kondensationsreaktion wird in Gegenwart einer Verbindung durchgeführt, die zur Deprotonierung einer Verbindung der allgemeinen Formel (II) befähigt ist. unter anderem werden bei des vorstehend beschriebenen synthese anorganische Basen wie Alkali-Alkoholate oder Carbonate, beispielsweise Kaliumcarbonat oder Natriumethanolat, eingesetzt. Die Reaktion wird in polaren Lösemitteln, wie beispielsweise Alkoholen, Ketonen oder Ethern, vorteilhaftesweise unter Zusatz von DMF bei Temperaturen von 90 bis 150°C, beispielsweise 110 bis 130°C für 10 bis 20, beispielsweise 12 bis 18 Stunden durchgeführt.

Darüber hinaus betrifft eine weitere Ausführungsform der vorstehend beschriebenen synthese Verfahren, bei dem die Verbindung R¹-X¹ durch eine Reaktion eines gerad- oder verzweigtkettigen, ein- oder mehrfach substituierten Alkans mit zwei Abgangsgruppen X¹ und X² und einem ein- oder mehrfach substituierten oder unsubstituierten Benzolderivat, beispielsweise eines Phenolderivats.

Diese Reaktion wird unter Bedingungen durchgeführt, die eine Reaktion des Benzolderivats, biespielsweise des Phenols, im Sinne einer Substitutionsreaktion erlauben, vorteilhaftesweise unter basischen Bedingungen. Die basischen Bedingungen können beispielsweise durch Zugabe eines Hydroxids, eines Alkoholats oder eines Carbonats, wie durch Zugabe von Natriumcarbonat oder Kaliumcarbonat, eingestellt werden. Vorteilhaftesweise wird das eingesetzte Alkan gleichzeitig als Lösemittel verwendet, gegebenenfalls unter Zusatz von DMF. Die Reaktion wird im Rahmen der vorstchend beschriebenen synthese bei Temperaturen von 100 bis 130°C, beispielsweise bei 115 bis 125 °C für 2 bis 8, beispielsweise 3 bis 6 Stunden durchgeführt.

Im Rahmen der vorstchend beschriebenen synthese sind solche Alkane vorteilhaft, die eine allgemeine Struktur X¹-(CR'R")ₙ-Zₘ-X² aufweisen, wobei
- R' und R" für Wasserstoff oder Fluor steht,
- Z für -P(CR'R")ₒ- mit P Sauerstoff oder Schwefel und o einer ganzen Zahl von 0 bis 4 steht, beispielsweise für 1 oder 2,
- X¹ und X² gute Abgangsgruppen sind, vorteilhafteweise unabhängig voneinander ausgewählt aus der Gruppe Halogen, Triflat, Tosylat, bespielsweise Chlor oder Brom,
- m eine ganze Zahl von 0 bis 1, beispielsweise 0 ist, und
- n eine ganze Zahl von 2 bis 8 ist, biespielsweise eine ganze Zahl von 4 bis 6.

Vorteilhaftig ist es im Rahmen der vorstehend beschriebenen synthese möglich, daß X¹ und X² identisch sind. Damit entstehen bei der Substitutionsreaktion keine Produktgemische, die nur aufwendig zu trennen sind. Beispielsweise wird im Rahmen der vorstehend beschriebenen synthese das Alkan im Überschuß eingesetzt. Das nicht abreagierte Alkan kann wieder zurückgewonnen werden.

In einer möglichen Ausführungsform der vorstehend beschriebenen synthese kann Epsilon-Caprolacton als Edukt eingesetzt werden, eine kostengünstig verfügbare Ausgangssubstanz mit geringer Toxizität. Epsilon-Caprolacton kann in verschiedene 1,6-bifunktionale Strukturen mit 6 Kohlenstoffatomen umgesetzt werden. Solche bifunktionalen Strukturen besitzen zwei verschiedene Substituenten, was es ermöglicht, selektiv eines der beiden Enden zu transformieren.

Um den Ring des Epsilon-Caprolactons zu öffnen, bieten sich mehrere Möglichkeiten an. Die Ringöffnung kann durch Amidierung unter Verwendung von beispielsweise Benzylamin (Lösungsmittel beispielsweise: Toluen, Tetrahydrofuran oder Xylen), Ethanolamin (vorzugsweise ohne zusätzliches Lösungsmittel, da das Ethanolamin selbst als Lösungsmittel fungiert), Dibutylamin oder Isopropylbenzylamin erfolgen, oder auch durch Veresterung oder nukleophile Ringöffnung, vorteilhaftesweise unter Verwendung von Bromwasserstoff, was zur Bildung von 6-Bromhexansäure führt.

Vorteilhafterweise erfolgt die Bildung von 6-Bromhexansäure durch Destillieren von Epsilon-Caprolacton in beispielsweise 48%iger Hydrobromsäure für ca. 3-4 Stunden. Anschließend wird das Reaktionsgemisch verdünnt und beispielsweise mit Toluen vermischt, bevor die saure Phase abgetrennt wird. Letzte Säurereste können beispielsweise mittels azeotroper Destillation entfernt werden. Nach abgeschlossener Destillation wird ein zweifacher Überschuss an Alkohol, beispielsweise Methanol, Ethanol oder n-Propanol, zugefügt und der Alkohol-Überschuss erneut durch Destillation abgetrennt

Sowohl die so erhaltenen Bromester als auch die freien Säuren können im Rahmen der vorstehend beschriebenen synthese eingesetzt werden.

Bei dem Benzolderivat handelt es sich vorsteilhaftesweise um ein Phenolderivat, biespielsweise um ein Phenol mit ein oder zwei weiteren Substituenten am aromatischen Ring. Günstige Substituenten sind beispielsweise Halogen, eine Alkyl- oder Alkoxygruppe, eine Nitrogruppe oder eine Trifluormethylgruppe, vorteilhaftesweise Chlor.

Es ist im Rahmen der vorstchend beschriebenen synthese auch möglich, daß die Verbindung der allgemeinen Formel (VIII) in eine Reaktion eingesetzt wird, bei der eine Verbindung der allgemeinen Formel (IX) entsteht, 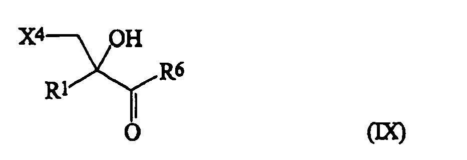 in der
- R¹ eine gerad- oder verzweigtkettige, ein- oder mehrfach substituierte oder unsubstituierte Alkylgruppe, eine gerad- oder verzweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist,
- X⁴ eine funktionelle Gruppe, die zur Ausbildung einer kationischen Zwischenstufe in einer Reaktion mit einer C-C-Doppelbindung befähigt ist und eine gute Abgangsgruppe ist, und
- R⁶ ausgewählt ist aus der Gruppe OH, O⁻M⁺, O⁻M²⁺, wobei M für ein Alkali-, Erdalkali- oder Erdmetall oder ein Kation einer organischen Stickstoffbase steht, und OR, wobei R für einen substituierten oder unsubstituierten Alkyl- oder Alkylenrest mit 1 bis 15 C-Atomen steht

Im Rahmen der vorstchend beschriebenen synthese wird R⁶ beispielsweise ausgewählt aus der Gruppe OH, O⁻Na⁺, O⁻K⁺, OR, wobei R für einen unsubstituierten Alkylrest mit 1 bis 10 C-Atomen steht.

Die Verbindung der allgemeinen Formel (IX) kann beispielsweise durch eine Hydrolysereaktion erhalten werden. Prinzipiell sind alle dem Fachmann bekannten Methoden anwendbar. Vorteilhaft ist die saure Hydrolyse unter Zugabe von Mineralsäuren wie beispielsweise Salzsäure oder Schwefelsäure. Erfindungsgemäß wird die Reaktion in polaren Lösemitteln wie Wasser oder Alkoholen, vorteilhaftesweise Wasser, bei Temperaturen über 80°C, beispielsweise bei 100°C für mindestens 24 Stunden, beispielsweise mindestens 36 Stunden, besonders günstig für mindestens 48 Stunden durchgeführt.

Es ist außerdem im Rahmen der vorstehend beschriebenen synthese möglich, daß die Verbindung der allgemeinen Formel (IX) in eine Reaktion eingesetzt wird, bei der ein Oxiran der allgemeinen Formel (X) 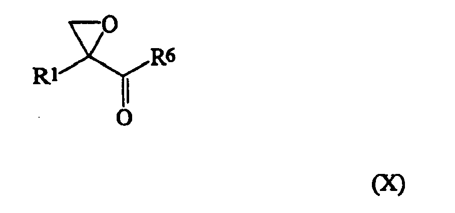 erhalten wird, in der
- R¹ eine gerad- oder verzweigtkettige, ein- oder mehrfach substituierte oder unsubstituierte Alkylgruppe, eine gerad- oder verzweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist, und
- R⁶ ausgewählt ist aus der Gruppe OH, O⁻M⁺, O⁻M²⁺, wobei M für ein Alkali-, Erdalkali- oder Erdmetall oder ein Kation einer organischen Stickstoffbase steht, und OR, wobei R für einen substituierten oder unsubstituierten Alkyl- oder Alkylenrest mit 1 bis 15 C-Atomen steht.

Die Reaktion zur Bildung des Oxirans findet unter Bedingungen statt, die den intramolekularen Angriff der OH-Gruppe im Sinne einer Substitutionsreaktion erlauben. Bei der vorstehend beschriebenen synthese läuft die Reaktion im basischen Bereich ab, wobei ein geeigneter pH-Wert beispielsweise durch Zugabe von Hydroxid oder Alkoholat, wie durch Zugabe von Kalium-tert-Butoxid, eingestellt wird. Die Reaktion kann in polaren Lösemitteln wie Wasser, Alkoholen, Ketonen, Ethern, bevorzugt MTBE, oder auch in Gemischen aus zwei oder mehr dieser Lösemittel ablaufen. Besonders günstig ist es auch, die Reaktion in einer getrockneten und konzentrierten Lösung eines unpolaren Lösemittels wie Toluol aus dem vorhergehenden Reaktionsschritt durchzuführen (siehe Beispiel 5). Die Reaktion wird im Rahmen der verstehend beschriebenen Synthese bei Temperaturen von -10 bis 10°C, beispielsweise bei Temperaturen unter 5°C, für 0,5 bis 2,5, beispielsweise 1 bis 2 Stunden, durchgeführt. Die Vollständigkeit der Reaktion kann beispielsweise durch HPLC überprüft werden.

Es ist im Rahmen der verstehend beschriebenen Synthese aber auch möglich, daß nach dem Ringschluß beispielsweise ein Salz einer Oxirancarbonsäure vorliegt, das dann in einem weiteren Reaktionsschritt in einen Carbonsäureester überführt wird, beispielsweise in einen Ester mit einem Alkohol mit 1 bis 15 Kohlenstoffatomen, beispielsweise mit 1 bis 10 Kohlenstoffatomen, besonders günstig mit 1 bis 6 Kohlenstoffatomen. Geeignete Reaktionsbedingungen für die Veresterung sind beispielsweise beschrieben in "Organikum", 18. Auflage, 1990, Deutscher Verlag der Wissenschaften Berlin, 400-408.

Die verstehend beschriebene Synthese behalt unteranderem ein Verfahren, das sich durch folgendes Ablaufschema beschreiben läßt, in dem die Reste R¹, R⁴, R⁵, R⁶, X¹, X², X⁴, Y¹ und Y² die oben genannte Bedeutung haben. R steht für H oder einen substituierten oder unsubstituierten Alkyl- oder Alkylenrest mit 1 bis 15 C-Atomen. Bei X³ handelt es sich unter anderem um einen Rest, der der Definition von L' entspricht, beispielsweise um ein Halogen, vorteilhaftesweise um Chlor:

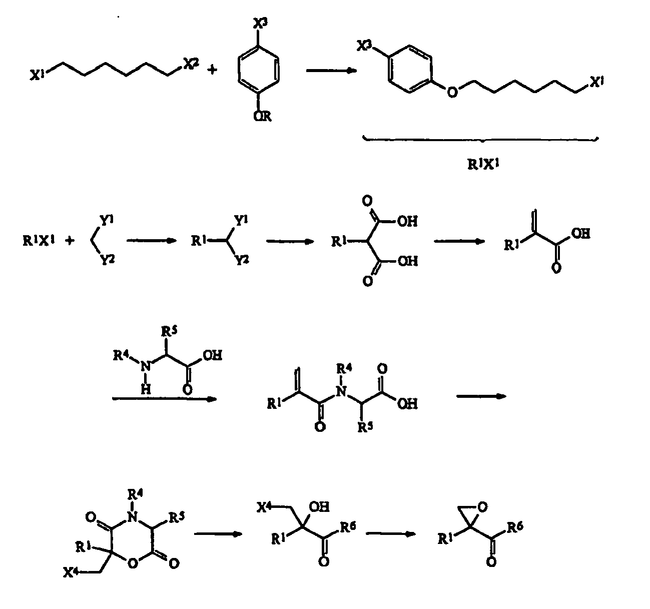

In der vorstehend beschriebenen Ausführungsform wird eine Oxirancarbonsäure, bzw. ein Derivat davon in acht Syntheseschritten ausgehend von leicht verfügbaren Edukten erhalten. Der letzte Reaktionsschritt ist der Ringschluß, so daß mit dem reaktiven Oxiran keine weiteren Reaktionsschritte durchgeführt werden müssen.

Des weiteren beschrieben ist ein Verfahren zur Herstellung von Oxirancarbonsäuren und Derivaten davon enthaltend die Synthese einer Verbindung der allgemeinen Formel (IX) enthaltend eine Reaktion einer Verbindung der allgemeinen Formel (VIII) zu der Verbindung der allgemeinen Formel (IX), 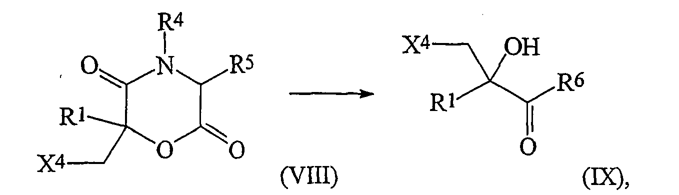 wobei
- R¹ eine gerad- oder verzweigtkettige, ein- oder mehrfach substituierte oder unsubstituierte Alkylgruppe, eine gerad- oder verzweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist,
- die Reste R⁴ und R⁵ für gleiche oder verschiedene gerad- oder verzweigtkettige, ein- oder mehrfach substituierte oder unsubstituierte Alkylgruppen, gerad- oder verzweigtkettige, ein- oder mehrfach substituierte oder unsubstituierte Alkylengruppen, gerad- oder verzweigtkettige, einoder mehrfach substituierte oder unsubstituierte Aralkyl-, Alkylaryl- oder Arylgruppen stehen, wobei R⁴NCR⁵ Teil einer substituierten oder unsubstituierten cyclischen Struktur, die auch ein weiteres Heteroatom ausgewählt aus der Gruppe N, S, O enthalten kann, sein kann,
- X⁴ eine funktionelle Gruppe, die zur Ausbildung einer kationischen Zwischenstufe in einer Reaktion mit einer C-C-Doppelbindung befähigt ist und eine gute Abgangsgruppe ist,
- nicht gleichzeitig R¹ für -(CH₂)₆-OBn steht und R⁴NCR⁵ für einen unsubstituierten Fünfring, und
- R⁶ ausgewählt ist OH, O⁻M⁺, O⁻M²⁺, wobei M für ein Alkali-, Erdalkalioder Erdmetall oder ein Kation einer organischen Stickstoffbase steht, oder OR, wobei R für einen substituierten oder unsubstituierten Alkyl- oder Alkylenrest mit 1 bis 15 C-Atomen steht.

Weitere Reaktionsschritte dieses Verfahrens können unter anderem die vorstehend näher beschriebenen Schritte sein.

Darüber hinaus behandelt die verstehend beschriebene Synthese auch ein Verfahren zur Herstellung von Oxirancarbonsäuren und Derivaten davon enthaltend die Synthese einer Verbindung der allgemeinen Formel (X) enthaltend eine Reaktion einer Verbindung der allgemeinen Formel (IX) bei der ein Oxiran der allgemeinen Formel (X) entsteht, 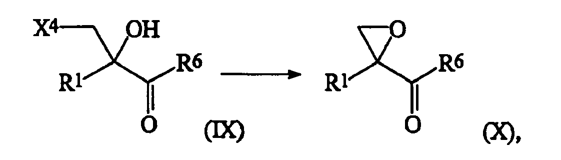 wobei
- R¹ eine gerad- oder verzweigtkettige, ein- oder mehrfach substituierte oder unsubstituierte Alkylgruppe, eine gerad- oder veizweigtkettige, einoder mehrfach substituierte oder unsubstituierte Alkylengruppe, eine gerad- oder verzweigtkettige, ein- oder mehrfach substituierte oder unsubstituierte Aralkyl-, Alkylaryl- oder Arylgruppe ist,
- R¹ nicht für -(CH₂)₆-OH steht,
- X⁴ eine funktionelle Gruppe, die zur Ausbildung einer kationischen Zwischenstufe in einer Reaktion mit einer C-C-Doppelbindung befähigt ist und eine gute Abgangsgruppe ist, und
- R⁶ ausgewählt ist aus OH, O⁻M⁺, O⁻M²⁺, wobei M für ein Alkali-, Erdalkali- oder Erdmetall oder ein Kation einer organischen Stickstoffbase steht, oder OR, wobei R für einen substituierten oder unsubstituierten Alkyl- oder Alkylenrest mit 1 bis 15 C-Atomen steht

Weitere Ausführungsformen dieses Verfahrens können die vorstehend näher beschriebenen Reaktionsschritte enthalten.

Gegenstand der vorliegenden Erfindung ist jedoch ein Verfahren zur Herstellung einer Oxirancarbonsäure und Derivaten davon, umfassend die Schritte (i) bis (vii)
(i) Umsetzung einer Verbindung der Struktur

   X¹-(CR'R")ₙ-Zₘ-X²

   wobei X¹ und X² unabhängig voneinander ausgewählt werden aus der Gruppe bestehend aus Halogen, Triflat und Tosylat, wobei n eine ganze Zahl von 2 bis 8 ist, wobei R' und R" für Wasserstoff oder Fluor oder einen Methylrest stehen, und wobei Z für Q(CR'R")ₒ mit Q gleich Sauerstoff oder Schwefel steht, m eine ganze Zahl von 0 bis 2 ist, o für eine ganze Zahl von 0 bis 4 steht, unter basischen Bedingungen mit einem Phenolderivat der Struktur 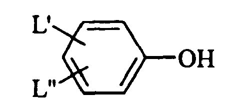 in der
   - L' und L" unabhängig voneinander für Wasserstoff, Halogen, eine substituierte oder unsubstituierte, verzweigte oder geradkettige Alkyl-, Aryl- oder Alkylarylgruppe, eine substituierte oder unsubstituierte, verzweigte oder geradkettige Alkoxy- oder Aryloxygruppe, eine substituierte oder unsubstituierte, verzweigte oder geradkettige Carboxyalkyl- oder Carboxyarylgruppe, eine Nitrogruppe oder eine Trifluormethylgruppe stehen, und
   unter Erhalt einer Verbindung der Struktur (I) 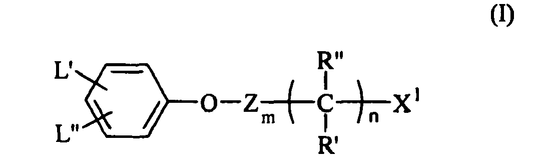
(ii) Umsetzung der gemäß (i) erhaltenen Verbindung der Struktur (I) mit einer Verbindung der Struktur (II) 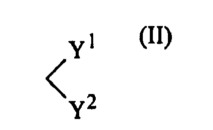 wobei Y¹ und Y² gleiche oder verschiedene elektronenziehende Gruppen sind, die in eine Carbonsäure umwandelbar sind,
   unter Erhalt einer Verbindung der Struktur (III) 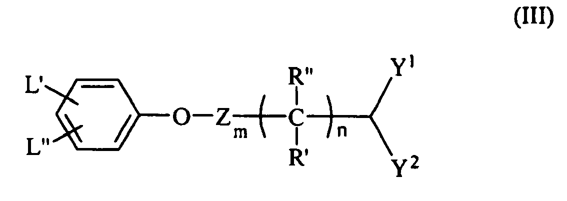
(iii) Hydrolyse der gemäß (ii) erhaltenen Verbindung der Struktur (III) zu einer Verbindung der Struktur (IV) 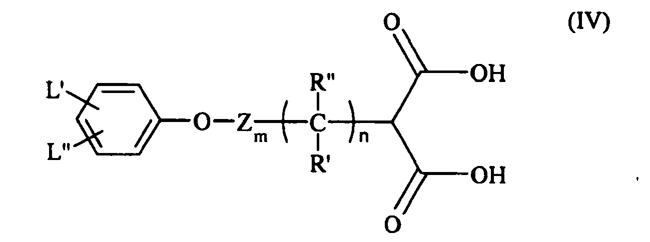
(iv) Umsetzung der gemäß (iii) erhaltenen Verbindung der Struktur (IV) zu einer Verbindung der Struktur (V) 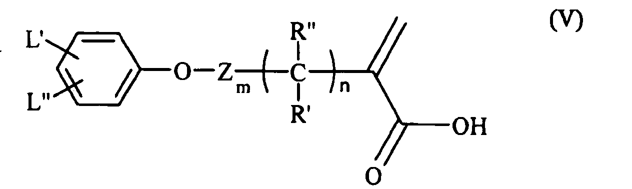
(v) Umsetzung der gemäß (iv) erhaltenen Verbindung der Struktur (V) mit einer Verbindung der Struktur (VI) 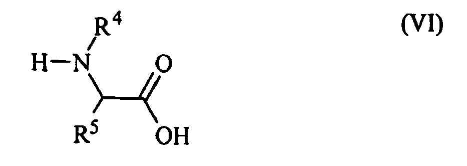 wobei R⁴ und R⁵ für gleiche oder verschiedene gerad- oder verzweigtkettige, einoder mehrfach substituierte oder unsubstituierte Alkylgruppen, gerad- oder verzweigtkettige, ein- oder mehrfach substituierte oder unsubstituierte Alkylengruppen, gerad- oder verzweigtkettige, ein- oder mehrfach substituierte oder unsubstituierte Aralkyl-, Alkylaryl- oder Arylgruppen stehen, wobei R⁴NCR⁵ Teil einer substituierten oder unsubstituierten cyclischen Struktur, die auch ein weiteres Heteroatom ausgewählt aus der Gruppe N, S, O enthalten kann, sein kann,
   wobei nicht gleichzeitig R⁴NCR⁵ für einen unsubstituierten Fünfring steht und 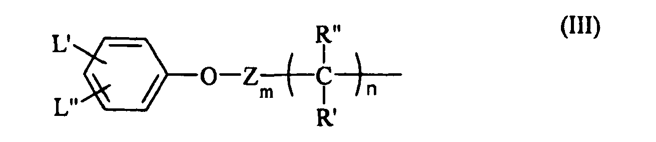 gleich -(CH₂)₆-OBn ist,
   unter Erhalt einer Verbindung der Struktur(VII) 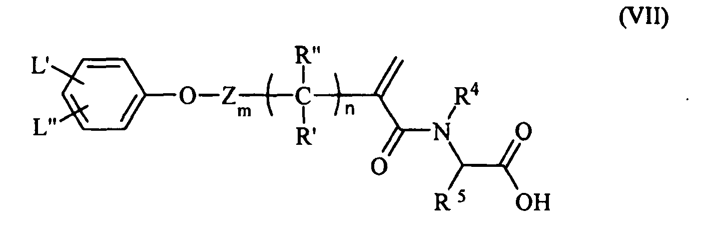
(vi) Umsetzung der gemäß (v) erhaltenen Verbindung der Struktur (VII) zu einer
   Verbindung der Struktur (VIII) 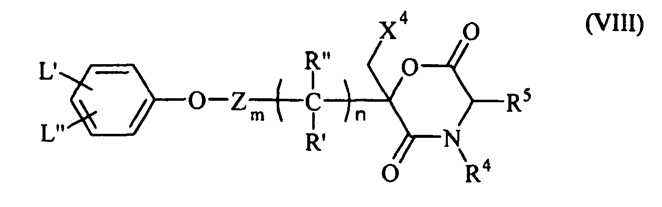 wobei X⁴ ein Halogen ist;
(vii) Hydrolyse der gemäß (vi) erhaltenen Verbindung der Struktur (VIII) zu einer Verbindung der Struktur (IX) 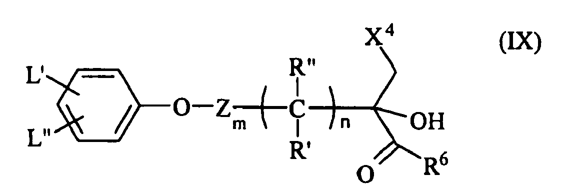 wobei R⁶ ausgewählt ist aus der Gruppe bestehend aus OH, O⁻M⁺, O⁻M²⁺ und OR, wobei M für ein Alkalimetall- oder Erdalkalimetallkation oder für ein Kation einer organischen Stickstoffbase steht und R für einen unsubstituierten oder substituierten Alkyl- oder Alkylenrest mit 1 bis 10 Atomen stehen.
(viii) Umsetzung der Verbindung der Struktur (IX) unter basischen Bedingungen zu einer Verbindung der Struktur (X)

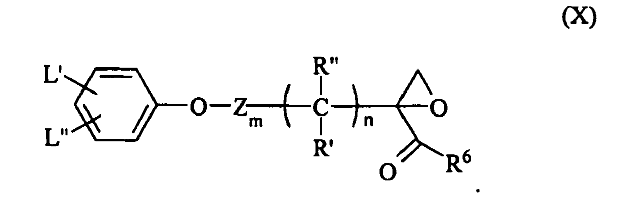

Darüber hinaus betrifft die vorliegende Erfindung auch ein Verfahren, in welchem Y¹ ein Rest der allgemeinen Struktur CO₂R² und Y² ein Rest der allgemeinen Struktur CO₂R³ ist, wobei R² und R³ unabhängig voneinander für einen ein- oder mehrfach substituierten oder unsubstituierten Alkylrest mit 1 bis 10 Kohlenstoffatomen stehen und gemäß Schritt (ii) die gemäß (i) erhaltene Verbindung mit einer Verbindung der speziellen Struktur (II) 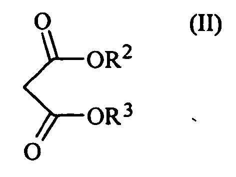 unter Erhalt einer Verbindung der speziellen Struktur (III) 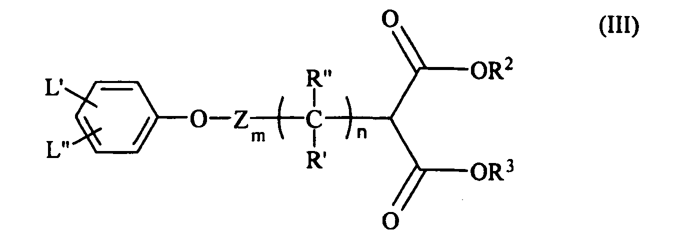 umgesetzt wird.

In einer bevorzugten Ausführungsform ist in dem erfindungsgemäßen Verfahren L' ein Halogen und L" gleich H.

In einer besonders bevorzugten Ausführungsform der vorliegenden Erfindung ist L' gleich Cl.

In einer weiteren Ausführungsform betrifft die vorliegende Erfindung auch ein Verfahren, in welchem R⁴ und R⁵ unabhängig voneinander für Alkylreste mit 1 bis 10 Kohlenstoffatomen stehen oder R4 und R5 Teil einer substituierten oder unsubsituierten cyclischen Struktur R⁴NCR⁵ sind.

Weiterhin betrifft die vorliegende Erfindung auch ein Verfahren, in welchem die in Schritt (v) eingesetzte Verbindung der Struktur (VI) Prolin oder ein Derivat davon ist.

Im Rahmen der vorliegenden Erfindung ist es auch möglich, daß ein erfindungsgemäßes Verfahren unter stereochemischer Kontrolle der einzelnen Reaktionsschritte abläuft.

Die erfindungsgemäß hergestellten Verbindungen können ein Chiralitätszentrum besitzen. Die Erfindung schließt daher sowohl die Racemate und die Enantiomeren als auch deren Gemische ein. Für die Racemattrennung werden dem Fachmann bekannte Methoden angewandt. Sofern das Verfahren unter stereochemischer Kontrolle abläuft, können die (+)-Enantiomere der Verbindungen hergestellt werden, beispielsweise durch Verwendung chiraler Hilfsreagenzien. Als chirale Hilfsreagenzien werden beispielsweise chirale Aminosäuren eingesetzt, so kann als Verbindung (VI) insbesondere L-Prolin eingesetzt werden. Es ist jedoch im Rahmen der vorliegenden Erfindung ebenso möglich, die (-)-Enantiomere herzustellen.

In einer weiteren Ausführungsform behandelt die verstehend beschriebene Synthese ein Verfahren zur Herstellung von Etomoxir, Palmoxirat oder Clomoxir, vorteilhafterweise von (+)-Etomoxir.

Daher betrifft die vorliegende Erfindung in einer weiteren Ausführungsform auch ein Verfahren, bei welchem die in Schritt (v) eingesetzte Verbindung der Struktur (VI) Prolin oder ein Derivat davon ist.

In einer besonders bevorzugten Ausführungsform betrifft die vorliegende Erfindung ein Verfahren, bei welchem die in Schritt (v) eingesetzte Verbindung der Struktur (VI) L-Prolin ist.

In einer weiteren Ausführungsform betrifft die vorliegende Erfindung auch ein Verfahren, bei welchem der Rest R⁶ ausgewählt ist aus der Gruppe bestehend aus OH, O⁻Na⁺, O⁻K⁺ und OR, wobei R für einen unsubstituierten Alkylrest mit 1 bis 10 Atomen steht.

Ein weiterer Gegenstand der Erfindung sind auch die nach einem erfindungsgemäßen Verfahren hergestellten Verbindungen, insbesondere die Oxirancarbonsäuren und Derivate davon, die nach den erfindungsgemäßen Verfahren herstellbar sind. Darüber hinaus sind auch Verbindungen der allgemeinen Formel (VII) beschrieben herstellbar gemäß Schritt (a) eines erfindungsgemäßen Verfahrens.

Die vorliegende Erfindung betrifft daher auch eine Verbindung der Struktur (VIII) 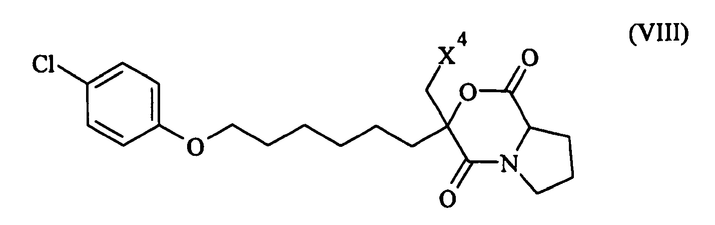 wobei X⁴ ein Halogen ist.

Bevorzugt ist in der erfindungsgemäßen Verbindung der Struktur (VIII) X⁴ gleich Brom.

Desweiteren betrifft die vorliegende Erfindung auch eine Verbindung der Struktur (IX) 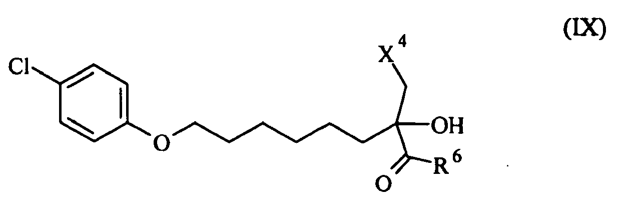 wobei X⁴ ein Halogen ist, und wobei R⁶ ausgewählt ist aus der Gruppe bestehend aus OH, O⁻M⁺, O⁻M²⁺ und OR, wobei M für ein Alkalimetall- oder Erdalkalimetallkation oder für ein Kation einer organischen Stickstoffbase steht und R für einen substituierten oder unsubstituierten Alkyl- oder Alkylenrest mit 1 bis 10 Atomen steht.

In einer bevorzugten Ausführungsform der vorliegenden Erfindung ist in der Verbindung der Struktur (IX) X⁴ gleich Br.

In einer weiteren bevorzugten Ausführungsform ist in der Verbindung der Struktur (IX) R⁶ ausgewählt aus der Gruppe bestehend aus OH, O⁻Na⁺, O⁻K⁺ und OR, wobei R für einen unsubstituierten Alkylrest mit 1 bis 10 Atomen steht.

Des weiteren beschrieben ist die Verwendung einer erfindungsgemäß hergestellten Oxirancarbonsäure oder eines Derivats davon in pharmazeutischen Zusam- mensetzungen, beispielsweise die Verwendung der erfindungsgemäß hergestellten Oxirancarbonsäuren und Derivaten davon zur Behandlung von Erkrankungen betreffend den Glucose-Stoffwechsel, den Fettstoffwechsel, von kardio-vaskulären Erkrankungen sowie der Herzinsuffizienz. Im einzelnen sind dies beispielsweise Diabetes, Hyperlipämie, koronare Herzerkrankungen wie Angina pectoris oder Myokardinfarkt, Zustand nach Myokardinfarkt oder dilatative Cardiomyopathien.

Nachfolgend wird die Erfindung anhand von beispielhaften Synthesen näher erläutert.

### BEISPIELE

### Beispiel 1

### 1. Bildung von R¹-X¹: 6-(4-Chlorophenoxy)hexylchlorid (6-CPHC) in 1,6-Dichlorhexan

Eingesetzte Substanzen:

| | | | |
|---|---|---|---|
| 4-Chlorphenol | 31,5 kg | 1 eq. | 245 Mol |
| 1,6-Dichlorhexan | 280 l | 7,9 eq. | 1,93 kMol |
| Kaliumcarbonat | 44 kg | 1,3 eq. | 318 Mol |
| DMF (Dimethylformamid) | 75 kg | | |
| n-Heptan | 200 kg | | |
| Wasser | 390 l | | |

4-Chlorphenol (als Schmelze) wurde zu 1,6-Dichlorhexan zugegeben, gefolgt von DMF. Kaliumcarbonat wurde zu dem Gemisch hinzugegeben. Das erhaltene viskose Gemisch wurde auf ca. 120°C erhitzt. Die Reaktion wurde für 3 - 6 Stunden fortgesetzt, bis HPLC-Kontrolle zeigte, daß der Anteil von 4-Chlorphenol unter 1 Flächen-% lag.

Wenn die Reaktion für vollständig befunden wurde, wurde das Reaktionsgemisch auf ca. 60 °C abgekühlt. Der Feststoff wurde abfiltriert. Der Filterkuchen wurde mit DMF (ca. 20 l) gewaschen.

Zu der vereinigten Mutterlauge und Waschlösung wurde Kaliumcarbonat (15 kg), n-Heptan (200 kg) und Wasser (235 l) hinzugefügt. Die Temperatur des erhaltenen Zwei-Phasen-Gemischs wurde auf 60 °C eingestellt und die wäßrige Phase wurde abgetrennt. Die organische Phase wurde mit Wasser (155 1) bei ungefähr 60 °C gewaschen.

Die verbleibende organische Phase wurde bei reduziertem Druck bei ca. 100 °C eingeengt. Wenn die Viskosität des Reaktionsprodukts ein Rühren nicht länger zuließ, wurde die Flüssigkeit in den nächsten Reaktionsschritt eingesetzt.

### 2. Kondensationsreaktion: Synthese von Diethyl-6-(4-chlorphenxy)hexyl-malonat (6-CPHMA-DEE) in Methanol

Eingesetzte Substanzen:

| 6-CPHC in 1,6-Dichlorhexan | 1/2 Ansatz aus der vorherigen Synthesestufe | | |
|---|---|---|---|
| Diethylmalonat | 28 l | 1,5 eq. | 186 Mol |
| Kalziumcarbonat | 3 5 kg | 2,1 eq. | 253 Mol |
| Kaliumbromid | 17 kg | 1,2 eq. | 143 Mol |
| Methanol | 80 l | | |
| DMF | 75 kg | | |
| 36%-ige Salzsäure | 5 l | | |
| n-Heptan | 135 kg | | |
| Wasser | 2251 | | |

Ein halber Reaktionsansatz des 6-CPHC in 1,6-Dichlorhexan wurde unter reduziertem Druck (<10 mbar) destilliert. Der Siedepunkt wurde vorzugsweise unter 130 °C gehalten. Das Destillat wurde auf den 1,6-Dichlorhexan-Gehalt analysiert und konnte für eine weitere Reaktion gemäß Schritt 1 eingesetzt werden.

Der Rückstand wurde auf 1,6-Dichlorhexan analysiert. Wenn der Gehalt unter 20 Flächen-% nach Gaschromatographie lag, wurde die Destillation beendet.

Nach beendeter Destillation wurde der Rückstand mit DMF verdünnt und in einen weiteren Reaktor überführt. Diethylmanolat, Kaliumcarbonat und Kaliumbromid wurden zu der DMF-Lösung hinzugegeben. Die erhaltene, viskose Mischung wurde auf etwa 130 °C erhitzt. Die Reaktionsmischung wurde für 12 -18 Stunden bei 130 °C gehalten, bis der Gehalt an Ausgangsmaterial unter 1 Flächen-% nach HPLC lag.

Das Reaktionsgemisch wurde auf 60 °C abgekühlt und der Feststoff abfiltriert. Der trockengesaugte Filterkuchen wurde mit DMF (30 1) gewaschen. n-Heptan (135 kg) und Wasser (150 l) wurden zu der Mutterlauge hinzugefügt. Die Mischung wurde auf ca. 40 °C erwärmt. Der pH-Wert wurde mit 36%-iger Salzsäure (ca. 5 l) auf einen pH=2 eingestellt. Die wäßrige Phase wurde abgetrennt und die organische Phase mit Wasser (751) gewaschen.

Nach Analyse konnten zwei Reaktionsansätze vereinigt werden. Die vereinigten Reaktionsansätze werden bei maximal 100 °C eingeengt. Gegen Ende des Einengens wurde der Druck reduziert, um soviel wie möglich des verbleibenden Diethylmalonats zu entfernen.

Methanol (80 l) wurde zu dem Rückstand hinzugegeben, um eine geeignete Konzentration für die nächste Reaktion zu erhalten.

### 3. Hydrolyse und Bildung der Doppelbindung: 8-(4-Chlorphenoxy)-2-methylenoctansäure (8-CPMOA)

Eingesetzte Substanzen:

| 6-CPHMA-DEE in Methanol | zwei vereinigte Ansätze aus Schritt 2 | | |
|---|---|---|---|
| Natriumhydroxid, 50%-Lösung | 114 kg | 6 eq. | 1,44 Mol |
| Methanol | 280 l | | |
| Wasser | 1030 l | | |
| n-Heptan | 675 kg | | |
| MTBE (Methyl-tert.butylether) | 280 l | | |
| 36%-ige Salzsäure | 163 1 | | |
| Isopropanol | 440 l | | |
| Paraformaldehyd | 12,5 kg | | |
| Piperidin | 29 l | | |

Die Natriumhydroxidlösung wurde zu dem Wasser/Methanol-Gemisch hinzugefügt. Die Temperatur der erhaltenen Mischung wurde auf 50 °C eingestellt. Zu dieser Mischung wurde 6-CPHMA-DEE in Methanol hinzugegeben. Die Geschwindigkeit der Zugabe wurde so eingestellt, daß die Temperatur unter 52 °C blieb. Die Zugabe wurde so schnell wie möglich durchgeführt. Nach beendeter Zugabe wurde Wasser (140 l) zugegeben. Nach der Wasserzugabe wurde die Reaktion als beendet betrachtet. Kleine Mengen der Ausgangsmaterialien konnten im weiteren Prozeß entfernt werden.

Methanol wurde im Vakuum bei maximal 50 °C entfernt. Während des Einengens wurde Wasser (190 l) zugegeben, um die Löslichkeit des Zwischenprodukts zu gewährleisten. n-Heptan (210 kg) und Wasser (100 l) wurden zugegeben und die Mischung auf 75 °C erhitzt. Die Wasserphase mit dem Produkt wurde abgetrennt und in einen anderen Reaktor überführt.

Die Temperatur der Wasserphase wurde unter 50°C abgesenkt, wonach MTBE (280 l) zugegeben wurden. Zu diesem Gemisch wurde 36%-ige Salzsäure (ca. 130 l) zugegeben, um den pH-Wert auf ca. 1 einzustellen. Während der Zugabe der Salzsäure wurde die Temperatur unter 40°C gehalten. Die wäßrige Phase wurde von der organischen Phase getrennt, die mit Wasser (190 l) bei 40 °C gewaschen wurde.

Isopropanol (290 l) wurde zu der organischen Phase hinzugegeben. Bei reduziertem Druck wurde MTBE bei maximal 40 °C entfernt. Die Destillation wurde fortgesetzt, bis ein Volumen von ca. 225 l im Reaktor verblieb.

Zu der erhaltenen Isopropanollösung wurde weiteres Isopropanol (150 l) und Paraformaldehyd (12,5 kg) hinzugefügt. Das erhaltene Gemisch wurde auf ca. 50°C erwärmt. Zu diesem Gemisch wurde Piperidin (29 l) so schnell wie möglich hinzugegeben, ohne daß die Temperatur 52 °C überschritt. Diese exotherme Reaktion resultierte in einem hochviskosen Reaktionsgemisch.

Die Reaktion wurde durch prozeßbegleitende HPLC-Analyse verfolgt. Als nach 1 - 2 Stunden weniger als 10% der freien Dicarbonsäure vorlagen, wurde zum Rückfluß erhitzt. Dies wurde solange durchgeführt, bis laut HPLC nur vernachläßigbare (<2%) Mengen des Intermediats vorlagen. Bei der Reaktion wurde Kohlenstoffdioxid freigesetzt. Eine typische Reaktionsdauer lag bei 3 Stunden. Während der Reaktion verringert sich die Viskosität des Reaktionsgemischs und gegen Ende lag eine fast klare Lösung vor.

Die Reaktion wurde als beendet betrachtet, wenn weniger als 5 Flächen-% (HPLC) des Zwischenprodukts vorlagen. Durch Einengen bei Atmosphärendruck wurde das Volumen im Reaktor auf ca. 160 l verringert. Zu dem Rückstand wurden Wasser (270 l) und n-Heptan (445 kg) zugegeben. 36%-ige Salzsäure (ca. 33 l) wurde zugegeben, bis ein pH-Wert von 1 erreicht war. Das erhaltene Zwei-Phasen-System wurde auf mindestens 70 °C erwärmt. Die Wasserphase wurde von der organischen Phase getrennt, die mit Wasser (140 l) bei mindestens 70 °C gewaschen wurde.

Das Volumen der organischen Phase wurde bei verringertem Druck und maximal 50 °C reduziert. Die Destillation wurde fortgeführt, bis ca. 490 1 im Reaktor verblieben. Gegen Ende der Destillation wurde eine Temperatur von 30 °C im Reaktor eingehalten. Nach vollständigem Einengen wird ein 8-CPMOA-Impfkristall hinzugegeben.

Der Reaktorinhalt wurde vorsichtig auf 0 °C abgekühlt.

Das Produkt wurde durch Filtration isoliert. Der Filterkuchen wurde mit kaltem n-Heptan (20 kg) gewaschen.

Nach Filtration konnte das Produkt ohne weitere Trocknung direkt in die nächste Reaktion eingesetzt werden. Pro Reaktionsansatz wurden ca. 37 - 40 kg 8-CPMOA erhalten.

### 4. Amidbildung: 8-CPMOA-Prolin

Eingesetzte Substanzen:

| 8-CPMOA | 40 kg (Trokkengewicht) | | 141 Mol |
|---|---|---|---|
| Natriumhydroxid, 50%-Lösung | 24 l | 3,00 eq. | 423 Mol |
| L-Prolin | 21 kg | 1,30 eq. | 182 Mol |
| Wasser | 85 l | | |
| Ethylacetat | 85 kg | | |
| Thionylchlorid | 14 l | 1,40 eq. | 198 Mol |
| Aceton | 38 kg | | |
| DMF | 106 kg | | |
| MTBE | 154 kg | | |
| 36%-ige Salzsäure | 25 l | | |
| Natriumchlorid | 25 kg | | |
| Wasser | 76 l | | |

Das durch Filtration erhaltene 8-CPMOA (ca. 40 kg Trockengewicht) wurde zu Ethylacetat (85 kg) hinzugegeben. Von der erhaltenen Lösung wurden ca. 19 1 Flüssigkeit unter reduziertem Druck bei maximal 40 °C eingedampft. DMF (1 l) wurde hinzugefügt und die Temperatur im Reaktor auf 45 °C eingestellt.

Thionylchlorid (14 l) wurde während 60 Min. hinzugegeben. Nach beendeter Zugabe wurde die Reaktion fortgeführt, bis HPLC zeigte, daß alles Ausgangsmaterial verbraucht worden war. Eine typische Reaktionszeit nach beendeter Zugabe lag bei 1 - 2 Stunden.

Durch Destillation bei maximal 45 °C wurde das Ethylacetat und das überschüssige Thionylchlorid entfernt. Aceton (38 kg) wurde hinzugefügt, und die erhaltene Mischung für mindestens 20 Min. gerührt, um eine vollständige Auflösung zu erhalten.

Natriumhydroxidlösung (24 l) und L-Prolin (21 kg) wurden zu Wasser (85 l) hinzugegeben. Während der Zugabe des L-Prolins wurde die Temperatur unter 20 °C gehalten. Zu diesem Gemisch wurde die Lösung des Säurechlorids in Aceton hinzugefügt, wobei die Temperatur unter 20 °C gehalten wurde. Nach beendeter Zugabe wurde die Reaktion fortgeführt, bis HPLC zeigte, daß alles Säurechlorid verbraucht worden war. Eine normale Reaktionszeit lag bei weniger als 30 Min..

Das Aceton wurde unter reduziertem Druck bei maximal 40 °C eingedampft. Es wurden insgesamt 48 1 Flüssigkeit eingedampft. MTBE (70 kg) wurde hinzugegeben und das erhaltene Zwei-Phasen-System wurde auf ca. 40 °C erwärmt. Die wäßrige Phase (das Produkt enthaltend) wurde abgetrennt. Zu der wäßrigen Phase wurde weiteres MTBE (84 kg) hinzugefügt, und die wäßrige Phase wurde mit 36%-iger Salzsäure (ca. 25 l) angesäuert. Der pH-Wert der Lösung wurde auf einen Wert von 1 - 2 eingestellt. Die Temperatur des Reaktorinhalts wurde auf 40 °C eingestellt und die wäßrige Phase abgetrennt. Die organische Phase wurde mit einer Mischung aus Wasser (76 l) und Natriumchlorid (25 kg) bei ca. 40 °C gewaschen.

Das MTBE wurde durch Destillation bei maximal 40 °C entfernt. Als der Rückstand eine ölige Konsistenz hatte, wurde DMF (105 kg) hinzugegeben, so daß die Lösung in den nächsten Reaktionsschritt eingesetzt werden konnte.

### 5. Lactonbildung: 8-CPMOA-Lacton

Eingesetzte Substanzen:

| 8-CPMOA-Prolin in DMF | 1 Ansatz aus dem vorherigen Reaktionsschritt | | |
|---|---|---|---|
| N-Bromsuccinimid (NBS) | 40 kg | 2,05 eq. | 289 Mol |
| Kalium-tert.-butoxid | 16 kg | 1,01 eq. | 143 Mol |
| Wasser | 1350 l | | |
| Natriumthiosulfat | 30 kg | | |
| Natriumcarbonat | 17kg | | |
| DMF | 177 kg | | |
| Ethanol | 400 l | | |
| MTBE | 570 l | | |
| Methanol | 510 l | | |

Es wurde eine Lösung von NBS (50 kg) in DMF (120 kg) hergestellt. Um nahezu alles NBS in Lösung zu erhalten, war es nötig, daß das DMF Raumtemperatur hatte.

Zu der Lösung von 8-CPMOA-Prolin in DMF aus dem vorhergehenden Reaktionsschritt wurde Kalium-tert.-butoxid bei einer Temperatur unter 5 °C zugegeben. Nach beendeter Zugabe lag der pH-Wert der Lösung über 7, anderenfalls wurde mehr Kalium-tert.-butoxid zugegeben.

Zu der Lösung von 8-CPMOA-Prolin wurde die Lösung von NBS zugegeben mit einer Geschwindigkeit, daß die Temperatur 0 °C nicht überschritt. Eine typische Zugabezeit lag zwischen 1 - 2 Stunden. Nach vollständiger Zugabe wurde die Temperatur auf 2 °C erhöht, wo sie für 1 Stunde gehalten wurde. Die Temperatur wurde auf 5 °C erhöht. Bei dieser Reaktionstemperatur wurde das Gemisch für ca. 12 - 17 Stunden gehalten. Während der Reaktion wurde ein weißlicher Feststoff gebildet.

Nach vollständiger Reaktion wurde die Reaktionslösung auf 0 °C abgekühlt. Der ausfallende Feststoff wurde durch Zentrifugation abgetrennt. Der Zentrifugationskuchen wurde mit DMF (57 kg) gewaschen. Zur Vervollständigung wurde eine Feinfiltration durchgeführt. Auf eine genaue Temperaturkontrolle der Mutterlauge unter 5 °C wurde streng geachtet. Der Feststoff wurde aufgrund der geringen thermischen Stabilität sofort in ausreichend Wasser suspendiert.

Wasser (230 l) und MTBE (570 l) wurden zu der Mutterlauge hinzugegeben. Die Zugabe von Wasser war exotherm, so daß die Temperatur unter 5 °C gehalten wurde. Eine Lösung von Natriumthiosulfat (30 kg) in Wasser (120 l) wurde hinzugegeben. Auch diese Zugabe war exotherm. Das Natriumthiosulfat zerstörte das verbleibende NBS, so daß die Temperatur nicht weiter kontrolliert werden mußte.

Das erhaltene Zwei-Phasen-System wurde für mindestens 30 Min. gerührt. Die untere Phase wurde dann abgetrennt. Wasser (490 l) und Natriumcarbonat (17 kg) wurden hinzugefügt und das Reaktionsgemisch auf 40 °C erwärmt. Die wäßrige Phase wurde abgetrennt. Die organische Phase wurde mit Wasser (510 l) gewaschen.

Die organische Phase wurde bei Atmosphärendruck eingeengt, bis ca. 170 l übrigblieben. Methanol (510 l) wurde hinzugegeben und das Eindampfen wurde fortgesetzt, bis der Siedepunkt 63 °C überstieg, d. h. bis alles MTBE verdampft war. Das Volumen im Reaktor wurde durch weiteres Einengen auf ca. 170 l eingestellt. Das Produkt wurde durch vorsichtiges Abkühlen der Lösung kristallisiert. Bei etwa 30 °C wurde die Lösung angeimpft, um die Kristallisation zu starten. Große Mengen an Impfkristallen waren nötig.

Um die Enantiomeren-Reinheit des Endprodukts zu garantieren, wurde eine Rekristallisation durchgeführt.

### Umkristallisation von 8-CPMOA-Lacton

Circa 50 kg (Trockengewicht) von 8-CPMOA-Lacton (dies entsprach 1 ½Batch-Ansätzen) wurde zu Ethanol (350 l) hinzugegeben. Durch Destillation bei Atmosphärendruck wurden ca. 100 1 Flüssigkeit abgedampft. Die Lösung wurde auf 45 °C abgekühlt, so daß die Lösung angeimpft werden konnte. Auch hier waren große Mengen von Impfkristallen nötig, um eine Kristallisation zu erreichen. Die Temperatur wurde langsam auf ca. 15 -18 °C abgesenkt.

Das Produkt wurde durch langsame Filtration isoliert und mit Ethanol gewaschen. Nach Filtration wurde das Produkt zunächst einige Stunden bei Raumtemperatur getrocknet. Die Temperatur wurde dann auf 40 °C erhöht. Die Ausbeute, die hier erreicht wurde, lag bei 42 kg pro Umkristallisation, was ca. 28 kg pro Syntheseansatz entsprach.

### 6. Freisetzung der Carbonsäure: 2-Bromoethyl-2-hydroxy-8-(4-chlorphenoxy)-octansäure (BH-COA)

Eingesetzte Substanzen:

| | | | |
|---|---|---|---|
| 8-CPMOA-Lacton | 42 kg | | 92 Mol |
| Toluol | 290 l | | |
| Wasser | 450 l | | |
| n-Heptan | 20 l | | |
| 36%-ige Salzsäure | 700 l | 92 eq. | 8400 Mol |

8-CPMOA-Lacton (42 kg) wurden in Wasser (180 l) gegeben. Zu der Suspension wurde 36%-ige Salzsäure (7001) gegeben.

Das Gemisch wurde zum Rückfluß erhitzt. Die Reaktion wurde für mindestens 24 Stunden, und bis zu 48 Stunden bei Rückflußtemperatur gehalten. Da beide Ausgangsmaterialien als Öl vorlagen, war es nötig, das Gemisch gut zu rühren.

Nach beendeter Reaktion wurde die Reaktionsmischung auf 75 °C abgekühlt und Toluol (270 l) wurde hinzugegeben. Die wäßrige Phase wurde abgetrennt und die Toluol-Phase wurde mit Wasser (270 l) bei 75 °C gewaschen.

Bei einer Temperatur unter 60 °C wurde Toluol abdestilliert, bis ein Volumen von ca. 200 l übrigblieb. Die Lösung wurde auf ca. 40 - 45 °C abgekühlt, so daß ein Impfkristall zugegeben werden konnte. Bei Einsetzen der Kristallisation wurde das Reaktionsgemisch auf etwa 55 °C erwärmt, und anschließend wurde durch Abkühlen kristallisiert. Das Abkühlen wurde bis zu einer Temperatur von 15 °C fortgesetzt.

Das Produkt wurde durch Filtration isoliert und anschließend mit Toluol (201) gewaschen. Der Filterkuchen wurde mit n-Heptan (20 l) gewaschen.

Das Rohprodukt konnte ohne Trocknen in den nächsten Reaktionsschritt eingesetzt werden. Eine typische Ausbeute lag bei ca. 24 kg BH-COA als berechnetes Trockengewicht.

### 7. Synthese von Etomoxir (Kaliumsalz)

Eingesetzte Substanzen:

| BH-COA | 16 kg (Trokkengewicht) | | 42 Mol |
|---|---|---|---|
| Kalium-tert.-butoxid | 10 kg | 2,11 eq. | 89 Mol |
| Wasser | | | |
| Methanol | 160 l | | |
| MTBE | 315 kg | | |
| Kaliumhydroxid | 3 kg | | |
| Wasser | 160 l | | |

BH-COA (16 kg als Trockengewicht) wurden zu MTBE (160 kg) zugegeben. Die erhaltene Lösung wurde auf ca. 0 - 5 °C abgekühlt. Zu diesem Gemisch wurde Kalium-tert.-butoxid mit einer Geschwindigkeit zugegeben, so daß die Temperatur unter 5 °C blieb. Eine typische Zugabezeit lag bei etwa 60 Min. Nach beendeter Zugabe wurde die Reaktion fortgesetzt (ca. 30 - 60 Min.), bis HPLC zeigte, daß die Ausgangsmaterialien abreagiert hatten.

Nach beendeter Reaktion wurde Wasser (160 1) zugegeben. Die wäßrige Phase (das Produkt enthaltend) wurde abgetrennt. MTBE (155 kg) wurden zu der wäßrigen Phase hinzugegeben, und der pH-Wert wurde mit 36%-iger Salzsäure auf einen Wert von 1 - 2 eingestellt. Die Temperatur des Systems wurde bei ca. 40 °C gehalten.

Die wäßrige Phase wurde abgetrennt und die organische Phase mit Wasser (1601) bei ca. 40 °C gewaschen.

MTBE wurde unter verringertem Druck bei ca. 40 °C abdestilliert, bis ein Volumen von ca. 100 l übrigblieb. Methanol (120 l) wurde hinzugegeben, und die Destillation bis zu einem Restvolumen von ca. 60 l 1 fortgesetzt.

Eine Lösung von Kaliumhydroxid (3 kg) in Methanol (301) wurde zu der methanolischen Lösung zugegeben. Während der Zugabe fiel das Produkt als Kaliumsalz aus. Nach beendeter Zugabe wurde das viskose Gemisch auf ca. 0 °C abgekühlt.

Das Produkt wurde durch Zentrifugation isoliert und anschließend mit Methanol (10 1) gewaschen. Das durch Zentrifugation erhaltene Produkt wurde bei 35 °C getrocknet. Eine typische Trockenausbeute lag bei ca. 12 kg.

### 8. Veresterung: Synthese (+)-Etomoxir

Eingesetzte Substanzen:

| | | | |
|---|---|---|---|
| Etomoxir, Kaliumsalz | 3,5 kg | | 10,4 Mol |
| Ethylbromid | 1,7 kg | 1,50 eq. | 15,6 Mol |
| n-Heptan | 39 l | | |
| Wasser | 44 l | | |
| Natriumcarbonat | 1,2 kg | | |
| DMF | 16 l | | |
| Ethanol | 400 l | | |

Ethylbromid (1,7 kg) wurde zu DMF (14 l) hinzugegeben. Die Mischung wurde auf 38 °C erwärmt. Etomoxir-Kaliumsalz wurde zu dem Gemisch mit einer Geschwindigkeit hinzugegeben, daß sich keine Klumpen bildeten.

Die Reaktion wurde bei 40 °C für ca. 18 - 20 Stunden fortgesetzt, bis HPLC zeigte, daß die Edukte verbraucht waren. Während der Reaktionszeit wurde das Reaktionsgemisch immer weniger viskos, und bei Ende der Reaktion lag nur noch ein leichter Niederschlag von Kaliumbromid vor.

Der Niederschlag wurde durch Filtration abgetrennt, und der Filterkuchen mit DMF (2 l) gewaschen. n-Heptan (141) und Wasser (14 l) wurden zu der Mutterlösung hinzugegeben. Das Zwei-Phasen-System wurde auf 50 °C erwärmt und die wäßrige Phase abgetrennt. n-Heptan (10 l) wurde zu der organischen Phase hinzugegeben. Die organische Phase wurde bei 50 °C zunächst mit Wasser (15 1) und Natriumcarbonat (1,2 kg) und anschließend mit Wasser (151) gewaschen.

10 l n-Heptan wurden bei maximal 50 °C unter verringertem Druck abdestilliert. Das Produktgemisch wurde durch einen Feinfilter abgesaugt. Der Feinfilter wurde anschließend mit n-Heptan (10 l) gespült.

14 l n-Heptan wurden bei maximal 50 °C unter verringertem Druck von der gefilterten Lösung abdestilliert. Die Lösung wurde auf ca. 19 - 20 °C abgekühlt, so daß ein Animpfen stattfinden konnte. Um das Bilden eines Öls zu verhindern, wurde während der Kristallisation mit hoher Geschwindigkeit gerührt. Nach dem Animpfen der Lösung wurde diese sehr vorsichtig bis auf ca. -5 °C abgekühlt.

Das Produkt wurde durch Filtration isoliert und mit 5 1 n-Heptan bei einer Temperatur unter 0 °C gewaschen. Das Produkt wurde sorgfältig trockengesaugt, da +-Etomoxir bei ca. 35 °C schmilzt und sehr vorsichtig behandelt werden mußte.

Das Produkt wurde im Vakuum bei maximal 20 °C getrocknet. Eine typische Ausbeute lag bei 2,4 kg (+)-Etomoxir.

### Beispiel 2

Die generelle Synthese der Oxirancarbonsäuren entsprach dem zuvor beschriebenen Beispiel 1. Allerdings wurde im ersten Reaktionsschritt die Menge an 1,6-Dichlorhexan von 7,9 auf 3 Äquivalente reduziert, um die Ausbeute des Reaktionsprozesses zu erhöhen und die Rohstoffkosten zu senken. Diese Modifikation resultierte zwar in einer vermehrten Entstehung des Nebenproduktes 1,6-Bis-(4-Chlorphenyl-)hexan, das jedoch in Schritt 3 leicht entfernt werden konnte. Zur weiteren Verarbeitung wurde Wasser zum Reaktionsgemisch hinzugefügt und das Produkt mittels MTBE extrahiert. Die ursprünglich notwendige Filtration wurde damit überflüssig.

### Beispiel 3

Auch in diesem Beispiel entsprach die generelle Synthese der Oxirancarbonsäuren dem in Beispiel 1 beschriebenen Reaktionsschema, ggf. mit der Änderung gemäß Beispiel 2. Als Ausgangssubstanz der Reaktion wurde jedoch anstelle von Kaliumcarbonat Natriumethoxid eingesetzt. Diese Modifikation hatte zur Folge, dass die Reaktion bereits nach 3-4 Stunden abgelaufen war und nicht wie zuvor erst nach 18 Stunden. Außerdem erforderte die Reaktion eine niedrigere Temperatur und hatte den Vorteil, dass weniger Nebenprodukte entstanden.

### Beispiel 4

Auch in diesem Beispiel entsprach die generelle Synthese der Oxirancarbonsäuren dem in Beispiel 1 beschriebenen Reaktionsschema, ggf. mit der Änderung gemäß Beispiel 2. Als Ausgangssubstanz der Reaktion wurde anstelle von Kaliumcarbonat Natriumethoxid gemäß Beispiel 3 eingesetzt. Bei der Lactonbildung gemäß Schritt 5 wurde das Endprodukt durch Umkristallisation in einem Gemisch aus Isopropanol und Heptan gereinigt, eine weitere Umkristallisation entfiel.

Bei der anschließenden Freisetzung der Carbonsäure gemäß Schritt 6 wurde 50%-ige Schwefelsäure anstelle von Salzsäure eingesetzt.

### Beispiel 5

Auch in diesem Beispiel entsprach die generelle Synthese der Oxirancarbonsäuren dem in Beispiel 1 beschriebenen Reaktionsschema, ggf. mit den Änderungen gemäß der Beispiele 2, 3 und/oder 4. Allerdings wurde 8-CPMOA-Lacton durch Erhitzen auf 125-130 °C für 8-12 Stunden in einer Mischung aus Wasser und Schwefelsäure erhitzt. Das Produkt wurde analog dem beschriebenen Verfahren isoliert, jedoch wurde die Toluollösung nach Trocknung und Konzentrierung mittels einer azeotropen Destillation ohne weitere Verarbeitung in dem nächsten Schritt verwendet. Hierdurch konnte die Bildung von chlorierten Nebenprodukten verhindert werden, was zu einer erhöhten Ausbeute, einer verkürzten Reaktionszeit und einem vereinfachten Verfahren führte.

Die so gewonnene Lösung wurde anschließend mit Kalium-tert.-butoxid behandelt. Die Aufarbeitung des Reaktionsproduktes erfolgte analog dem beschriebenen Verfahren, jedoch wurde anstelle der Salzsäure verdünnte Phosphorsäure verwendet. Für die Präzipitation des Produktes wurde n-Propanol als Lösemittel und anstelle von Kaliumhydroxid in Methanol eine wässrige Lösung von Kaliumkarbonat verwendet. Anschließend wurde die n-Propanollösung des Kaliumsalzes des Reaktionsproduktes erhitzt, bis eine beinahe klare Lösung erhalten wurde. Das Produkt wurde durch Filtration bei oder unterhalb Raumtemperatur gesammelt und schließlich getrocknet. Anschließend wurde das Produkt aus Ethanol rekristallisiert. Diese Änderungen des letzten Verfahrensschrittes führten zu verbesserten Filtrationseigenschaften des Materials, was letztendlich auch zu einer erhöhten Produktqualität führte.

## Patentansprüche

1. Verfahren zur Herstellung einer Oxirancarbonsäure und Derivaten davon, umfassend die Schritte (i) bis (vii)
(i) Umsetzung einer Verbindung der Struktur
X¹-(CR'R")ₙ-Zₘ-X²
wobei X¹ und X² unabhängig voneinander ausgewählt werden aus der Gruppe bestehend aus Halogen, Triflat und Tosylat, wobei n eine ganze Zahl von 2 bis 8 ist, wobei R' und R" für Wasserstoff oder Fluor oder einen Methylrest stehen, und wobei Z für Q(CR'R")ₒ mit Q gleich Sauerstoff oder Schwefel steht, m eine ganze Zahl von 0 bis 2 ist, o für eine ganze Zahl von 0 bis 4 steht, unter basischen Bedingungen
mit einem Phenolderivat der Struktur 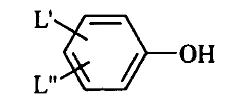 in der
- L' und L" unabhängig voneinander für Wasserstoff, Halogen, eine substituierte oder unsubstituierte, verzweigte oder geradkettige Alkyl-, Aryl- oder Alkylarylgruppe, eine substituierte oder unsubstituierte, verzweigte oder geradkettige Alkoxy- oder Aryloxygruppe, eine substituierte oder unsubstituierte, verzweigte oder geradkettige Carboxyalkyl- oder Carboxyarylgruppe, eine Nitrogruppe oder eine Trifluormethylgruppe stehen, und
unter Erhalt einer Verbindung der Struktur (I) 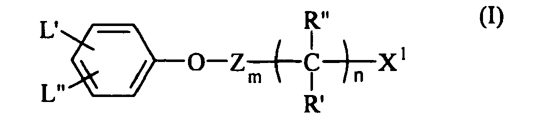
(ii) Umsetzung der gemäß (i) erhaltenen Verbindung der Struktur (I) mit einer Verbindung der Struktur (II) 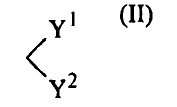 wobei Y¹ und Y² gleiche oder verschiedene elektronenziehende Gruppen sind, die in eine Carbonsäure umwandelbar sind,
unter Erhalt einer Verbindung der Struktur (III) 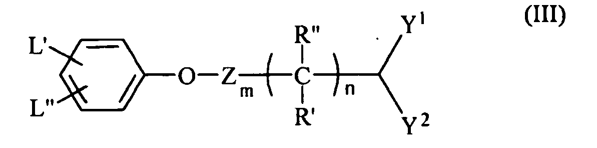
(iii) Hydrolyse der gemäß (ii) erhaltenen Verbindung der Struktur (III) zu einer Verbindung der Struktur (IV) 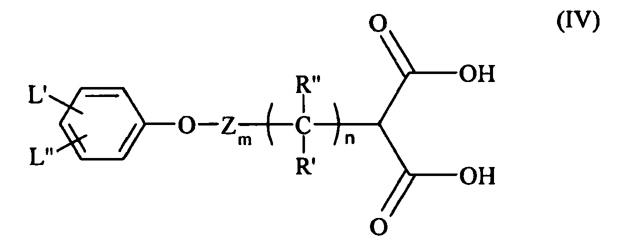
(iv) Umsetzung der gemäß (iii) erhaltenen Verbindung der Struktur (IV) zu einer Verbindung der Struktur (V) 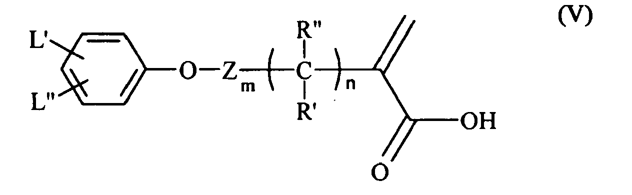
(v) Umsetzung der gemäß (iv) erhaltenen Verbindung der Struktur (V) mit einer Verbindung der Struktur (VI) 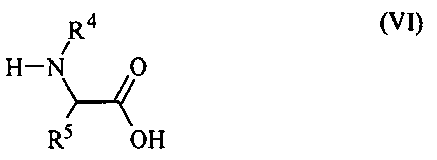 wobei R⁴ und R⁵ für gleiche oder verschiedene gerad- oder verzweigtkettige, ein- oder mehrfach substituierte oder unsubstituierte Alkylgruppen, gerad- oder verzweigtkettige, ein- oder mehrfach substituierte oder unsubstituierte Alkylengruppen, gerad- oder verzweigtkettige, ein- oder mehrfach substituierte oder unsubstituierte Aralkyl-, Alkylaryl- oder Arylgruppen stehen, wobei R⁴NCR⁵ Teil einer substituierten oder unsubstituierten cyclischen Struktur, die auch ein weiteres Heteroatom ausgewählt aus der Gruppe N, S, O enthalten kann, sein kann,
wobei nicht gleichzeitig R⁴NCR⁵ für einen unsubstituierten Fünfring steht und 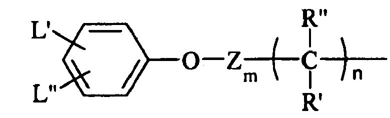 gleich -(CH₂)₆-OBn ist,
unter Erhalt einer Verbindung der Struktur (VII) 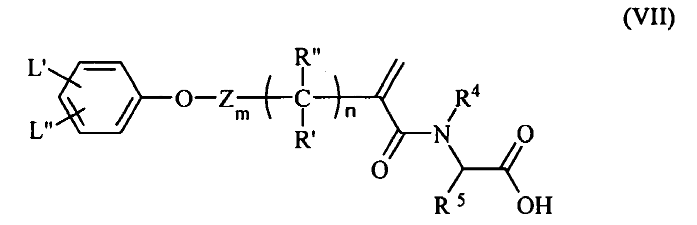
(vi) Umsetzung der gemäß (v) erhaltenen Verbindung der Struktur (VII) zu einer Verbindung der Struktur (VIII) 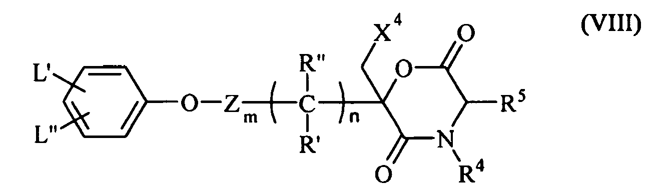 wobei X⁴ ein Halogen ist;
(vii) Hydrolyse der gemäß (vi) erhaltenen Verbindung der Struktur (VIII) zu einer Verbindung der Struktur (IX) 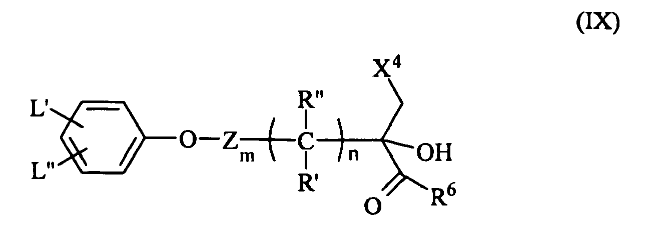 wobei R⁶ ausgewählt ist aus der Gruppe bestehend aus OH, O⁻M⁺, O⁻M²⁺ und OR, wobei M für ein Alkalimetall- oder Erdalkalimetallkation oder für ein Kation einer organischen Stickstoffbase steht und R für einen unsubstituierten oder substituierten Alkyl- oder Alkylenrest mit 1 bis 10 Atomen stehen.
(viii) Umsetzung der Verbindung der Struktur (IX) unter basischen Bedingungen zu einer Verbindung der Struktur (X)

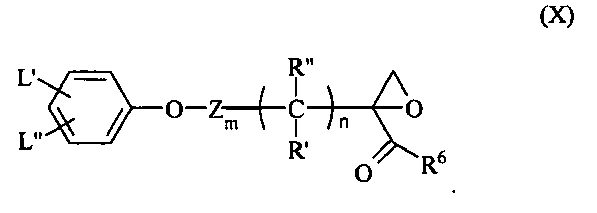

2. Verfahren nach Anspruch 1, **dadurch gekennzeichnet, dass** Y¹ ein Rest der allgemeinen Struktur CO₂R² und Y² ein Rest der allgemeinen Struktur CO₂R³ ist, wobei R² und R³ unabhängig voneinander für einen ein- oder mehrfach substituierten oder unsubstituierten Alkylrest mit 1 bis 10 Kohlenstoffatomen stehen und gemäß Schritt (ii) die gemäß (i) erhaltene Verbindung mit einer Verbindung der speziellen Struktur (II) 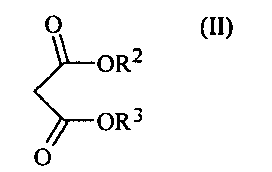 unter Erhalt einer Verbindung der speziellen Struktur (III) 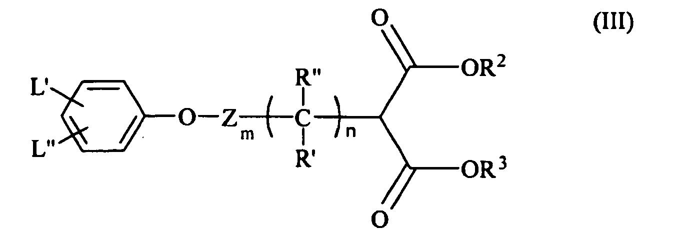 umgesetzt wird.

3. Verfahren nach Anspruch 1 oder 2, **dadurch gekennzeichnet, dass** L' ein Halogen ist und L" gleich H ist.

4. Verfahren nach Anspruch 3, **dadurch gekennzeichnet, dass** L' gleich Cl ist.

5. Verfahren nach einem der Ansprüche 1 bis 4, **dadurch gekennzeichnet, dass** R⁴ und R⁵ unabhängig voneinander für Alkylreste mit 1 bis 10 Kohlenstoffatomen stehen oder R⁴ und R⁵ Teil einer substituierten oder unsubstituierten cyclischen Struktur R⁴NCR⁵ sind.

6. Verfahren nach einem der Ansprüche 1 bis 5, **dadurch gekennzeichnet, dass** die in Schritt (v) eingesetzte Verbindung der Struktur (VI) Prolin oder ein Derivat davon ist.

7. Verfahren nach Anspruch 6, **dadurch gekennzeichnet, dass** die in Schritt (v) eingesetzte Verbindung der Struktur (VI) L-Prolin ist.

8. Verfahren nach einem der Ansprüche 1 bis 7, **dadurch gekennzeichnet, dass** R⁶ ausgewählt ist aus der Gruppe bestehend aus OH, O⁻Na⁺, O⁻K⁺ und OR, wobei R für einen unsubstituierten Alkylrest mit 1 bis 10 Atomen steht.

9. Verfahren nach einem der Ansprüche 1 bis 8, **dadurch gekennzeichnet, dass** m gleich 0 und R' und R" gleich H sind.

10. Verbindung der Struktur (VIII) 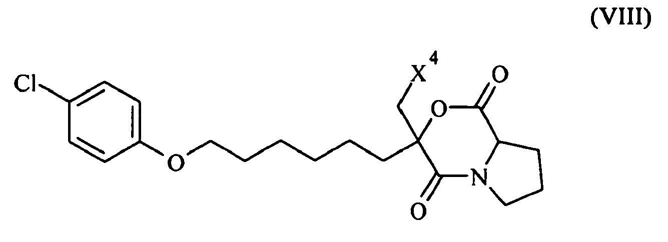 wobei X⁴ ein Halogen ist.

11. Verbindung nach Anspruch 10, **dadurch gekennzeichnet, dass** X⁴ gleich Br ist.

12. Verbindung der Struktur (IX) 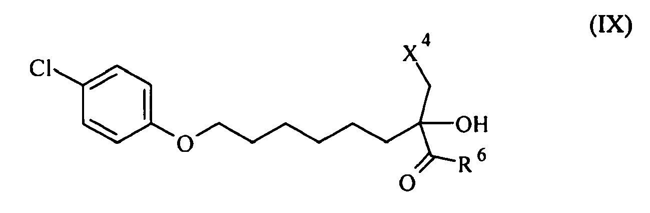 wobei X⁴ ein Halogen ist, und wobei R⁶ ausgewählt ist aus der Gruppe bestehend aus OH, O⁻M⁺, O⁻M²⁺ und OR, wobei M für ein Alkalimetall- oder Erdalkalimetallkation oder für ein Kation einer organischen Stickstoffbase steht und R für einen substituierten oder unsubstituierten Alkyl- oder Alkylenrest mit 1 bis 10 Atomen steht.

13. Verbindung nach Anspruch 12, **dadurch gekennzeichnet, dass** X⁴ gleich Br ist.

14. Verbindung nach Anspruch 12 oder 13, **dadurch gekennzeichnet, dass** R⁶ ausgewählt ist aus der Gruppe bestehend aus OH, O⁻Na⁺, O⁻K⁺ und OR, wobei R für einen unsubstituierten Alkylrest mit 1 bis 10 Atomen steht.

## Claims

1. A method for the production of an oxirane carboxylic acid and derivatives thereof, comprising the steps (i) to (vii)
(i) reacting a compound of the structure
X¹-(CR'R")ₙ-Zₘ-X²
wherein X¹ and X² are independently from each other selected from the groups consisting of halogen, triflate and tosylate, wherein n is an integer from 2 to 8, wherein R' and R" represent hydrogen or fluor or a methyl residue, and wherein Z represents Q(CR'R")ₒ with Q being oxygen or sulfur, m is an integer from 0 to 2, o represents an integer from 0 to 4, under basic conditions
with a phenol derivative of the structure 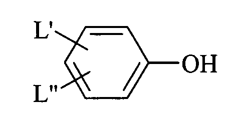 in which
- L' and L" independently from each other represent hydrogen, halogen, a substituted or unsubstituted, branched or linear alkyl, aryl or alkylaryl group, a substituted or unsubstituted, branched or linear alkoxy or aryloxy group, a substituted or unsubstituted, branched or linear carboxyalkyl or carboxyaryl group, a nitro group or a trifluormethyl group, and
yielding a compound of the structure (I) 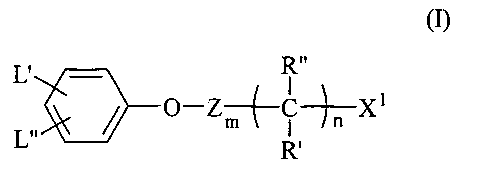
(ii) reacting the compound of the strucure (I) obtained according to (i) with a compound of the structure (II) 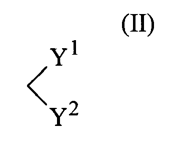 wherein Y¹ and Y² are the same or different electron-withdrawing groups, which are convertible to a carboxylic acid,
yielding a compound of the structure (III) 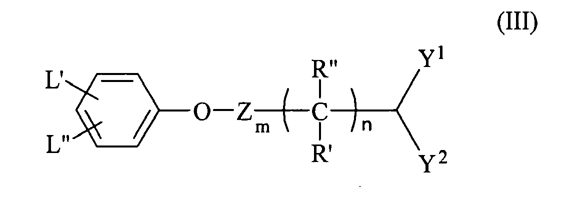
(iii) hydrolysis of the compound of the structure (III) obtained according to (ii) to a compound of the structure (IV) 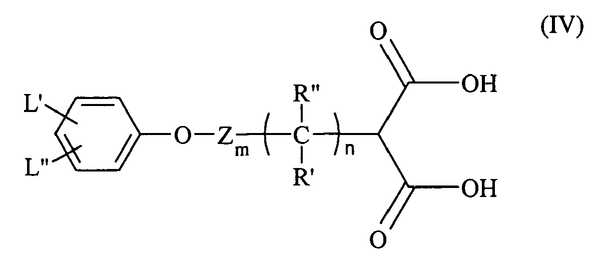
(iv) reacting the compound of the structure (IV) obtained according to (iii) to a compound of the structure (V) 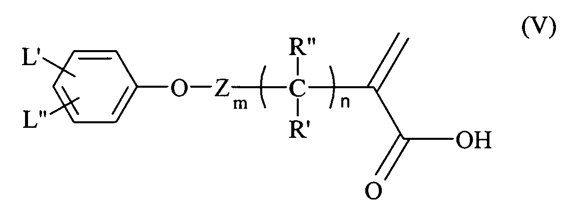
(v) reacting the compound of the structure (V) obtained according to (iv) to a compound of the structure (VI) 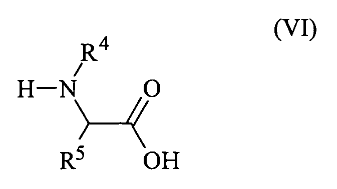 wherein R⁴ and R⁵ represent the same or different linear or branched, singly or multiply substituted or unsubstituted alkyl groups, linear or branched, singly or multiply substituted or unsubstituted alkylene groups, linear or branched, singly or multiply substituted or unsubstituted aralkyl, alkylaryl or aryl groups, wherein R⁴NCR⁵ may be part of a substituted or unsubstituted cyclic structure, which may also contain a further hetero atom selected from the group N, S, O,
wherein not simultaneously R⁴NCR⁵ represents an unsubstituted five-membered ring and 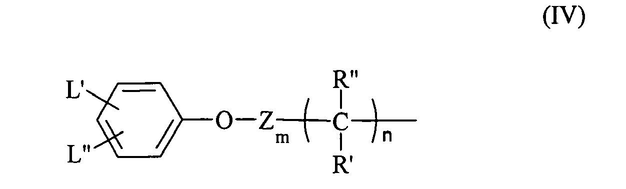 is -(CH₂)₆-OBn,
yielding a compound of the structure (VII) 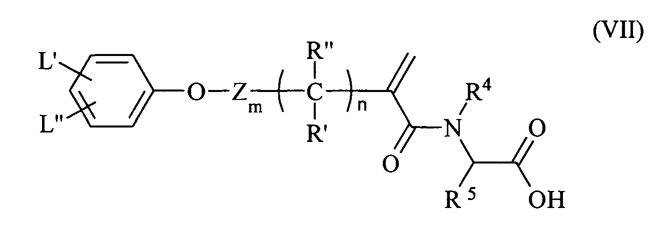
(vi) reacting the compound of the structure (VII) obtained according to (v) to a compound of the structure (VIII) 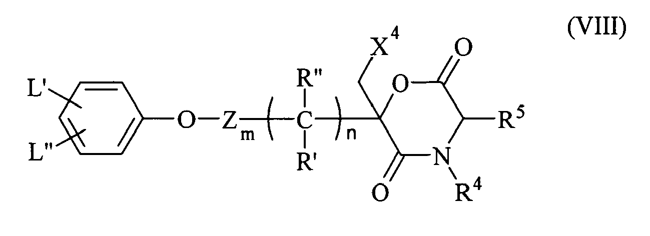 wherein X⁴ is a halogen;
(vii) hydrolysis of the compound of the structure (VIII) obtained according to (vi) to a compound of the structure (IX) 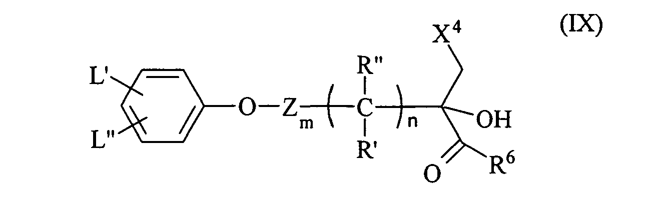 wherein R⁶ is selected from the group consisting of OH, O⁻M⁺, O⁻M²⁺ and OR, wherein M represents an alkali metal or alkaline earth metal cation or a cation of an organic nitrogenous base and R represents an unsubstituted or substituted alkyl or alkylene residue with 1 to 10 atoms,
(viii) reacting the compound of the structure (IX) under basic conditions to a compound of the structure (X)

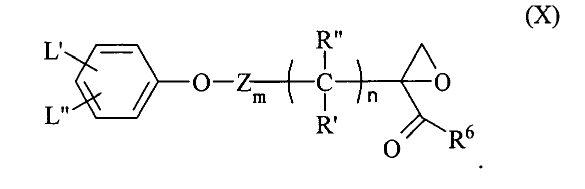

2. The method of claim 1, **characterized in that** Y¹ is a residue of the general structure CO₂R² and Y² is a residue of the general structure CO₂R³, wherein R² and R³ independently from each other represent a singly or multiply substituted or unsubstituted alkyl residue with 1 to 10 carbon atoms and according to step (ii) the compound obtained according to (i) is reacted with a compound of the specific structure (II) 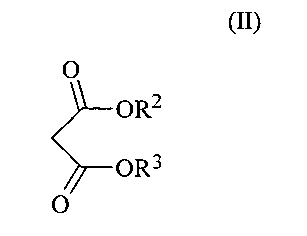 yielding a compound of the specific structure (III)

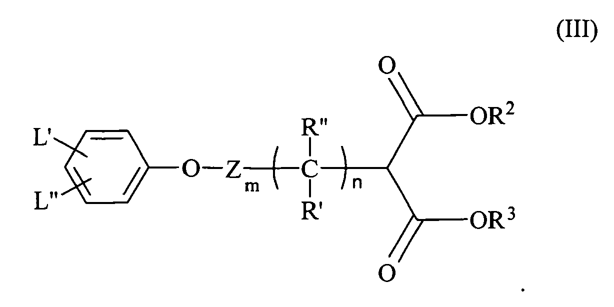

3. The method of claim 1 or 2, **characterized in that** L' is a halogen and L" is H.

4. The method of claim 3, **characterized in that** L' is Cl.

5. The method of any one of claims 1 to 4, **characterized in that** R⁴ and R⁵ independently from each other represent alkyl residues with 1 to 10 carbon atoms or R⁴ and R⁵ are part of a substituted or unsubstituted cyclic structure R⁴NCR⁵.

6. The method of any one of claims 1 to 5, **characterized in that** the compound of the structure (VI) applied in step (v) is prolin or a derivative thereof.

7. The method of claim 6, **characterized in that** the compound of the structure (VI) applied in step (v) is L-prolin.

8. The method of any one of claims 1 to 7, **characterized in that** R⁶ is selected from the group consisting of OH, O⁻Na⁺, O⁻K⁺ and OR, wherein R represents an unsubstituted alkyl residue with 1 to 10 atoms.

9. The method of any one of claims 1 to 8, **characterized in that** m is 0 and R' and R" are H.

10. A compound of the structure (VIII) 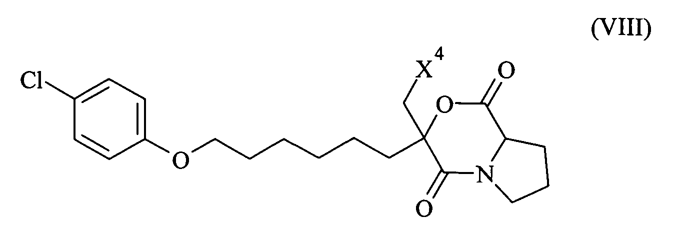 wherein X⁴ is a halogen.

11. The compound of claim 10, **characterized in that** X⁴ is Br.

12. A compound of the structure (IX) 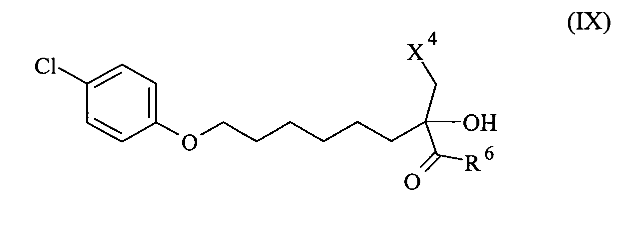 wherein X⁴ is a halogen, and wherein R⁶ is selected from the group consisting of OH, O⁻M⁺, O⁻M²⁺ and OR, wherein M represents an alkali metal or alkaline earth metal cation or a cation of an organic nitrogenous base and R represents a substituted or unsubstituted alkyl or alkylene residue with 1 to 10 atoms.

13. The compound of claim 12, **characterized in that** X⁴ is Br.

14. The compound of claim 12 or 13, **characterized in that** R⁶ is selected from the group consisting of OH, O⁻Na⁺, O⁻K⁺ and OR, wherein R represents an unsubstituted alkyl residue with 1 to 10 atoms.

## Revendications

1. Procédé de préparation d'un acide carboxylique oxirane et de ses dérivés, comprenant les étapes (i) à (viii)
(i) la réaction d'un composé de formule
**X¹-(CR'R")ₙ-Zₘ-X²**
dans lequel X¹ et X² sont choisis chacun indépendamment dans le groupe comprenant un atome d'halogène, un groupe triflate et un groupe tosylate, dans lequel n est un nombre entier de 2 à 8, dans lequel R' et R" représentent un atome d'hydrogène, de fluor ou un radical méthyle, et dans lequel Z représente Q(CR'R")ₒ , où Q représente également un atome d'oxygène ou un atome de soufre, m est un nombre entier de 0 à 2, o représente un nombre entier de 0 à 4, dans des conditions basiques,
avec un dérivé phénolique de formule 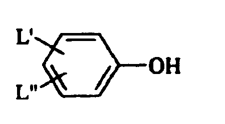 dans laquelle L' et L" indépendamment représentent un atome d'hydrogène, un atome d'halogène, un groupe alkyle, aryle, ou arylalkyle substitué ou non substitué, ramifié ou linéaire, un groupe alcoxy ou aryloxy substitué ou non substitué, ramifié ou linéaire, un groupe carboxyalkyle ou carboxyaryle substitué ou non substitué, ramifié ou linéaire, un groupe nitro ou un groupe trifluorométhyle,
pour l'obtention d'un composé de formule (I) 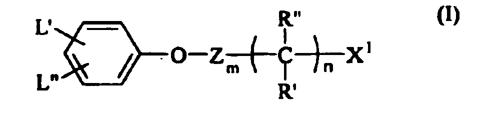
(ii) la réaction du composé obtenu selon l'étape (i) de formule (I) avec un composé de formule (II) 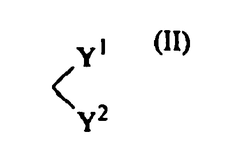 dans laquelle Y¹ et Y² sont des groupes électroattracteurs identiques ou différents, qui sont susceptibles d'être transformés en acide carboxylique
pour l'obtention d'un composé de formule (III) 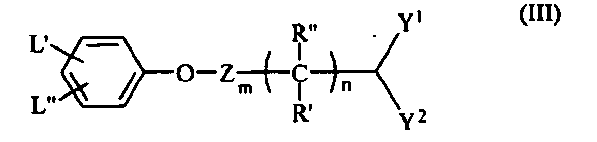
(iii) l'hydrolyse du composé obtenu selon l'étape (ii) de formule (III) en un composé de formule (IV) 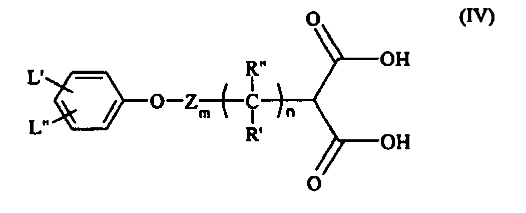
(iv) la conversion du composé obtenu selon l'étape (iii) de formule (IV) en un composé de formule (V) 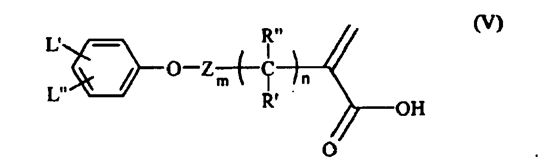
(v) la réaction du composé obtenu selon l'étape (iv) de formule (V) avec un composé de formule (VI) 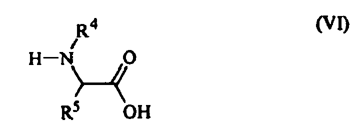 dans laquelle R⁴ et R⁵ sont identiques ou différents et sont des groupes alkyles linéaires ou ramifiés, mono ou pluri-substitués ou non substitués, des groupes alkylènes linéaires ou ramifiés, mono ou pluri-substitués ou non substitués, des groupes aralkyles, alkylaryles, aryles, linéaires ou ramifiés, mono ou pluri-substitués, ou non substitués,
dans laquelle le groupe R⁴NCR⁵ peut faire partie d'une structure cyclique substituée ou non substituée, qui peut en outre comprendre un hétéroatome supplémentaire choisi parmi le groupe N, S, O
dans laquelle, alternativement, le groupe R⁴NCR⁵ représente un cycle à cinq atomes non substitué et 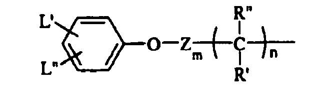 -(CH₂)₆-OBn,
pour l'obtention d'un composé de formule (VII) 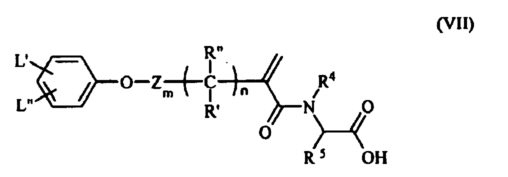
(vi) la conversion du composé de formule (VII) obtenu selon l'étape (v) en un composé de formule (VIII) 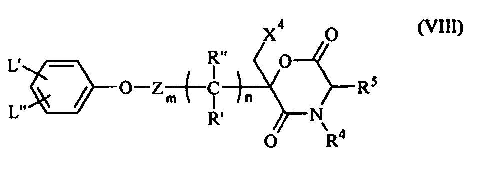 dans laquelle X⁴ est un halogène ;
(vii) l'hydrolyse du composé de formule (VIII) obtenu selon l'étape (vi) en un composé de formule (IX) 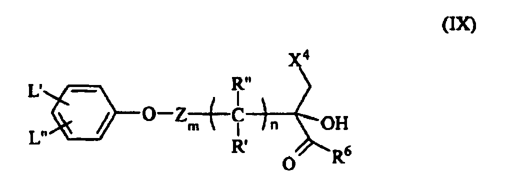 dans laquelle R⁶ est choisi dans le groupe comprenant OH, O⁻M⁺, O⁻M²⁺ et OR, où M représente un cation métallique alcalin ou un cation métallique alcalino-terreux, ou un cation d'une base organique nitrique et R représente un radical alkyl ou alkylène non substitué ou substitué, de 1 à 10 atomes ;
(viii) conversion du composé de formule (IX) dans des conditions basiques pour obtenir le composé de formule (X)

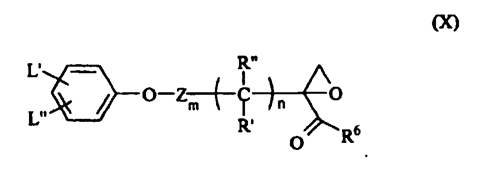

2. Procédé selon la revendication 1, **caractérisé en ce que** Y¹ est un radical de formule générale CO₂R² et Y² est un radical de formule générale CO₂R³, où R² et R³ représentent indépendamment un radical alkyle mono ou pluri-substitué ou non substitué, de 1 à 10 atomes de carbone et selon l'étape (ii), le composé obtenu selon l'étape (i) est mis en réaction avec un composé de formule particulière (II) 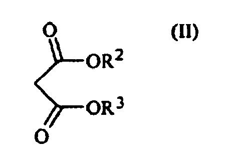 pour l'obtention d'un composé de formule (III)

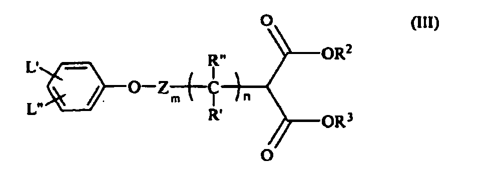

3. Procédé selon la revendication 1 ou la revendication 2, **caractérisé en ce que** L' est un atome d'halogène et L" est H.

4. Procédé selon la revendication 3, **caractérisé en ce que** L' est Cl.

5. Procédé selon l'une quelconque des revendications 1 à 4, **caractérisé en ce que** R⁴ et R⁵ sont indépendamment un radical alkyle de 1 à 10 atomes de carbone, ou bien R⁴ et R⁵ font partie d'une structure cyclique R⁴NCR⁵ substituée ou non substitué.

6. Procédé selon l'une quelconque des revendications 1 à 5, **caractérisé en ce que** à l'étape (v), le composé utilisé de structure (VI) est la Proline ou un de ses dérivés.

7. Procédé selon la revendication 6, **caractérisé en ce que** à l'étape (v), le composé utilisé de structure (VI) est la L-Proline.

8. Procédé selon l'une quelconque des revendications 1 à 7, **caractérisé en ce que** R⁶ est choisi dans le groupe comprenant OH, O⁻Na⁺, OK⁺ et O⁻R où R représente un radical alkyle non substitué de 1 à 10 atomes.

9. Procédé selon l'une quelconque des revendications 1 à 8, **caractérisé en ce que** m est O et que R' et R" sont H.

10. Composé de formule (VIII) 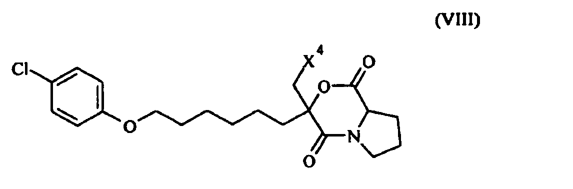 dans laquelle X⁴ est un halogène.

11. Composé selon la revendication 10, **caractérisé en ce que** X⁴ est Br.

12. Composé de formule (IX) 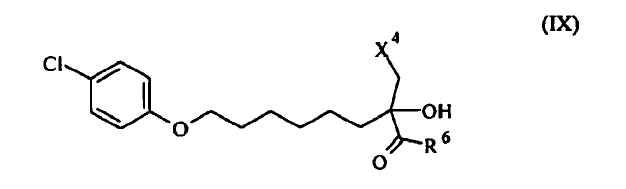 dans laquelle X⁴ est un atome d'halogène, et dans laquelle R⁶ est choisi dans le groupe comprenant OH, O⁻M⁺, O⁻M²⁺ et OR où M représente un cation métallique alcalin ou un cation métallique alcalino-terreux, ou un cation d'une base organique nitrique et R représente un radical alkyle ou alkylène substitué ou non substitué de 1 à 10 atomes.

13. Composé selon la revendication 12, **caractérisé en ce que** X⁴ est Br.

14. Composé selon la revendication 12 ou la revendication 13, **caractérisé en ce que** R⁶ est choisi dans le groupe comprenant OH , O⁻Na⁺, O⁻K⁺ et OR, où R représente un radical alkyle non substitué de 1 à 10 atomes.
